(19) Europäisches Patentamt
European Patent Office
Office européen des brevets

(11) Publication number: 0 267 996 A1

# (12) EUROPEAN PATENT APPLICATION

(21) Application number: **86309090.8**

(22) Date of filing: **20.11.86**

(51) Int. Cl.4: **C07H 19/06**, C07H 19/16, C07H 19/20, C07H 21/00

(43) Date of publication of application: **25.05.88 Bulletin 88/21**

(84) Designated Contracting States: **AT BE CH DE ES FR GB GR IT LI LU NL SE**

(71) Applicant: **TAMIR BIOTECHNOLOGY LTD.**
**P.O. Box 20192**
**Tel Aviv, 61201(IL)**

(72) Inventor: **Segev, David**
**Moshav Bene Reem 40**
**Doar Na Evtah 79840(IL)**

(74) Representative: **Kosmin, Gerald Emmanuel et al**
**HASELTINE, LAKE & CO. Hazlitt House 28 Southampton Buildings Chancery Lane**
**London, WC2A 1AT(GB)**

## (54) New nucleotide derivatives.

(57) Nucleotide derivatives useful in modified DNA probe synthesis are compounds having the formula (I) wherein either A is a radical of formula $-NHCO-(CH_2)_x-R^3$ and B is a substituted pyrimidine or purine residue, or A is a hydrogen atom and B is a radical of formula (II)

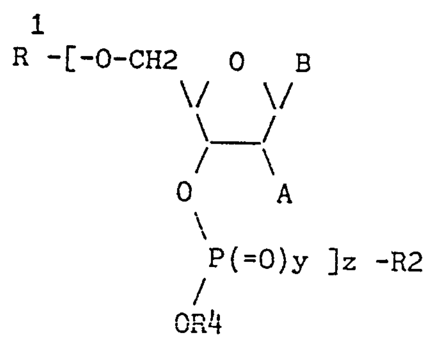

(I)

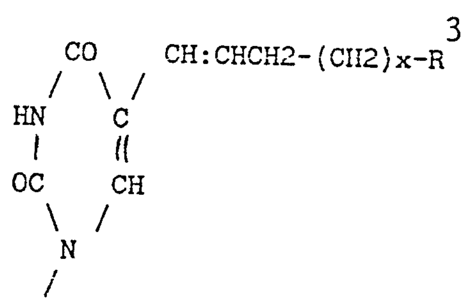

(II)

The terminal group $R^3$ may comprise amino or carboxy groups, or a sugar moiety, or it may comprise a reporter group such as biotin, or luminol derivatives, or any other group which is detectable by Elisa or

EP 0 267 996 A1

other means of detection. These compounds do not exist in natural DNA. The new compounds are useful as active monomers in a rapid and efficient procedure for the synthesis of DNA fragments and can be chemically incorporated in a DNA probe synthesis which allows total control over the extent and site of modification.

# NEW NUCLEOTIDE DERIVATIVES

The present invention relates to new nucleotide derivatives and their use in modified DNA probe synthesis. More particularly, this invention relates to the preparation and use of such novel compounds, in which there has been attached to one of two alternative positions of a nucleotide, which is further derivatized in the phosphorus-containing group, either an -NHCO-or and $-CH:CHCH_2$-group, to which a terminal group, preferably with the interposition of a carbon chain of up to twenty-one carbon atoms, is in turn attached. The terminal group may comprise for example amino or carboxy groups, or a sugar moiety, or it may comprise a reporter group such as biotin, or luminol derivatives, or any other group which is detectable by Elisa or other means of detection. These compounds do not exist in natural DNA.

The new compounds are useful as active monomers in a rapid and efficient procedure for the synthesis of DNA fragments and can be chemically incorporated in a DNA probe synthesis which allows total control over the extent and site of modification.

Oligonucleotides have thus been synthesized, incorporating monomers having side chains including a non-radioactive reporter group, as well as with monomers having side chains terminating in an amine or carboxy group, which could then be selectively modified by attachment of non-radioactive reporter groups, such as biotin, nitroiodophenyl acetic acid, dansyl or fluorescein in excellent yields.

## BACKGROUND OF THE INVENTION

Deoxyribonucleic acid (DNA) probes - small pieces of DNA that "recognize" specific genes - make it possible to identify and isolate the genetic information of any organism. Originally developed as basic tools for genetic engineering research, DNA probes are now beginning to enter the market as reagents for infectious disease and microbiological testing by hybridization techniques.

At the present time, nucleic acid hybridization assays are used primarily in the field of molecular biology as a research tool for the detection and identification of a unique deoxyribonucleic acid (DNA) sequence or specific gene in a complete DNA, a mixture of DNA's or mixture of DNA fragments. A number of variations of the technique exist and are used in recombinant DNA research. (See Methods in Enzymology, Vol. 68, R. Wu (Ed.) pp. 379-469, 1979; and Dunn, A.R. and Sambrook, J., Methods in Enzymology, Vol. 65, Part 1, pp. 468-478, 1980.)

Many procedures employed in diagnostics rely on the use of nucleotide or polynucleotide derivatives radioactively labelled with phosphorus($^{32}P$), iodine($^{125}I$) or sulfur($^{35}S$). Such radioactive probes provide useful tools for the detection, monitoring or isolation of compounds containing genetic information, even when present in extremely minute amounts.

However, there are serious limitations and drawbacks associated with the use of radioactive probes. Firstly, since personnel who handle radioactive material can be exposed to potentially hazardous levels of radiation, elaborate safety precautions must be maintained during the preparation, utilization and disposal of the radioisotopes. Secondly, radioactive nucleotides are extremely expensive to purchase and use, in large part due to the cost of the equipment and manpower necessary to provide the appropriate safeguards, producer/user health monitoring services, and waste-disposal regimens. Thirdly, radioactive materials are often very unstable and have a limited shelf-life, which further increases usage costs. This instability results from radiolytic decomposition, due to the destructive effects associated with the decay of the radioisotope itself, and from the fact that many isotopes (e.g. $^{32}P$and $^{125}I$) have half-lives of only a few days. Radioactive probes have been used in the detection of genetic targets using hybridization techniques.

One of the most widely used procedures is called the Southern blot filter hybridization method [Southern, E., J. Mol. Biol. 98: 503 (1975)]. This procedure is usually used to identify a particular DNA fragment separated from a mixture of DNA fragments by electrophoresis. The procedure is generally carried out by isolating a sample of DNA from some organism. The isolated DNA is subjected to restriction endo-nuclease digestion and electrophoresed on a gel (agarose, acrylamide, etc.). When the gel containing the separated DNA fragments is put in contact (blotted) with a nitrocellulose filter sheet (or diazotized paper, etc.), the fragments are transferred and become bound to the nitrocellulose sheet. The gel-transfer nitrocellulose sheet containing the DNA fragments is then heated (about 95°C) to denature the DNA. At this point, the sheet is treated with a solution containing denatured radiolabelled ($^{32}P$) "specific DNA probe" and hybridization is allowed to take place. Hybridization can take from 3 to 48 hours, depending on given conditions. Unhybridized "specific DNA probe" is then washed away. The nitrocellulose sheet is placed on a sheet of X-ray film which is exposed by the radioactive material, which usually takes several days at

-70°C. The X-ray film is then developed. The exposed areas on the film identify which DNA fragments have hybridized and therefore have a sequence similar to that of the "specific DNA probe".

New technologies for the detection of a DNA target provide the basis for much commercial research and development. It is now possible to label DNA with enzymes, fluorescent molecules, or chemiluminescent catalysts, rather than conventional radioisotopes. See, for example, European Patent 0070685 to M. Heller. However, incorporation of most of these label materials is a slow, complicated process.

Present methods exist by which a biotinylated deoxyuridine triphosphate is incorporated into the DNA probe by nick translation [Pennina R. Langer, Alex A. Waldrop and David C. Ward, Proc. Natnl. Acad. Sci. USA 78: 6633 (1981)]. This biotinylated DNA probe is used to detect specific DNA and RNA sequences in fixed cells and tissue sections following in situ hybridization and visualization on nitrocellulose membranes. The subsequent detection of the DNA probe is accomplished by either fluorescent antibody or enzymatic detection.

The disadvantage of this method is the dependence of the biotinylated probe on the clean target, which requires laborious work for the detection of the correct target, such as a gene, virus or any DNA target cloned into a host cell like a plasmid, phage, etc.

When the target DNA is screened to get a clean clone, biotinylated deoxyuridine triphosphate is incorporated by nick translation using DNA polymerase in order to get a new modified DNA probe which is denatured later to separate the modified DNA probe. This biotinylated DNA probe is now ready to detect the same target in a new organism by hybridization technique.

An alternative approach is to prepare the whole probe, including deoxyuridine modified at C-5, by chemical synthesis using phosphoramidite methodology. See Matteuci, M.D. and Caruthers, M.H., Tetrahedron Letters 21: 3243 (1980) and Matteuci, M.D. et al, J. Am. Chem. Soc. 103: 3185 (1981).

The hydrolytic stability of these phosphoramidites is dramatically demonstrated by their high-yield preparation in a reaction sequence which includes an aqueous workup. A further advantage of phosphoramidite chemistry is that little or no 3'-3' dimer is formed.

Chemical synthesis of modified nucleosides including substituted uracil and cytosine nuclear analogs with a "linker arm" of four to nineteen atoms in length attached at C-5 and terminating in chemically reactive functionalities (primary amines, carboxylic acids) and including a chlorine modified phosphoridite substituent, is generally described by Jerry L. Ruth in "Chemical Synthesis of Non-Radioactively-Labeled DNA Hybridization Probes", published in DNA 3, No. 1, 1984.

There is a definite need in the area of clinical diagnostics for a simple and rapid method for detecting and identifying unique nucleotide (genome) sequences, in very minute quantities. For example, many so-called "slow infection" diseases of human beings and animals, where symptoms appear long after the infectious process is initiated, are caused by viruses or by virus-like agents. These diseases include Herpes simplex I and II, cytomegalovirus, Kuru, and subacute sclerosing panacephalitis, for example. These slow infection diseases cannot be detected by immunodiagnostic techniques because no viral antigens are present. Therefore, hybridization assays are used to directly detect the viral genome [A.T. Hasse et al, Science, 21L: 672-674 (1981).

Hybridization assays would also be useful in determining common animal diseases, such as piglet diarrhea, which kills 20 million animals annually. Probes could also be used to study hybridization in plants. They are useful to help visualize the crossed traits without growing the plant to full capacity.

A further potential human application of DNA probes is detecting genes which can cause disease. For example, they could be adapted to prenatal diagnosis, making detection for abortion possible in all genetic diseases. Thus, hybridization could play an important role in any case where low antigenic response or lack thereof precludes the use of immunodiagnostic techniques.

The disadvantages associated with the use of radioactive probes have been noted above. The present invention provides novel compounds which may be used in non-radioactive probes, thus overcoming such disadvantages. These probes can be used for the detection and diagnosis of diseases in an economical, rapid and reliable manner, as will be described hereinbelow.

## SUMMARY OF THE INVENTION

The present invention accordingly provides a chemical compound having the formula (I) wherein either A is a radical of formula $-NHCO-(CH_2)_x-R^3$ and B is a substituted pyrimidine or purine residue, or A is a hydrogen atom and B is a radical of formula (II)

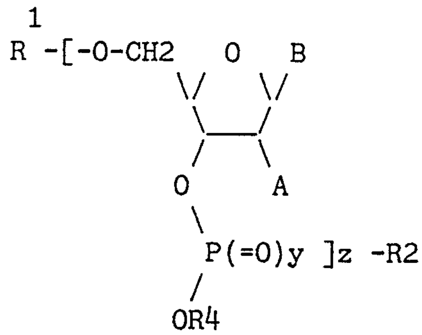

(I)

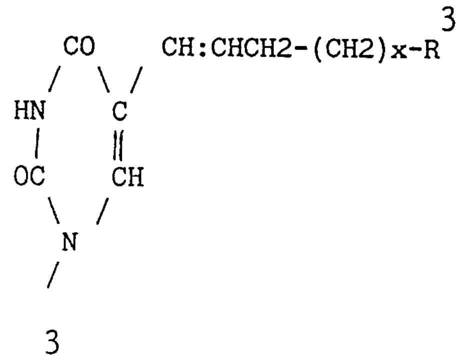

(II)

x is 0-21, $R^3$ is selected from the group consisting of $NH_2$, acylamido, biotinamido, dansyl, alkoxycarbonyl, carboxyl, carbamoyl, substituted carbamoyl, thiocarbamoyl, substituted thiocarbamoyl, ureido, substituted ureido, thioureido, substituted thioureido, a furanoside sugar radical, a pyranoside sugar radical and other reporter groups which can be detected without a requirement for radioactive labelling; $R^1$ is a hydrogen atom, a labile group removable by acid or a radical of formula (III);

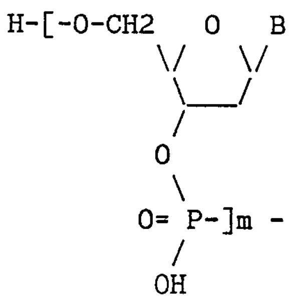

(III)

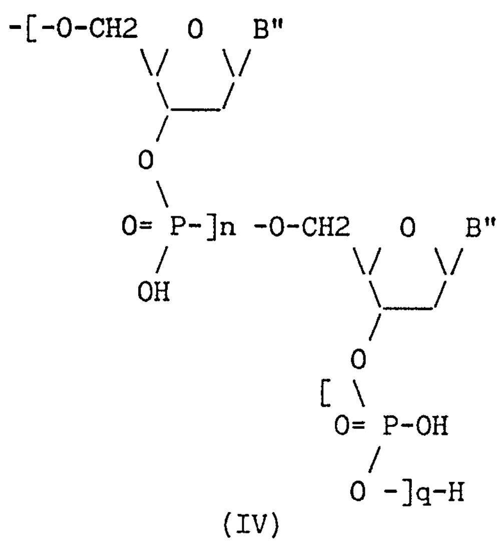

(IV)

$R^2$, when $R^1$ is a hydrogen atom or a labile group removable by acid, is halogen, NR′NR″ (in which R′ and

R″ are the same or different alkyl radicals, or NR′R″ forms a saturated heterocyclic ring) or tetrazolyl, or $R^2$, when $R^1$ is is a radical of formula (III), is a radical of formula (IV); $R^4$, when $R^1$ is hydrogen atom or a labile group removable by acid, is alkyl, or $R^4$, when $R^1$ is is a radical of formula (III), is a hydrogen atom; B′ and B″ are each the same or different substituted pyrimidine or purine residues; m and n are each 0 or an integer of 1 to about 1000, q and y are independently 0 or 1, and z is 1 when $R^1$ is a hydrogen atom or a labile groupe removable by acid, or otherwise z is 1 to about 100; provided that when $R^1$ is a radical of formula (III) and $R^2$ is a radical of formula (IV), then the compound of formula (I) is a modified deoxyribonucleic acid (or a corresponding oligonucleotide) in which 1 to about 100 monomeric units in any position(s) have been replaced by the same number of units of formula (V),

```
-O-CH2   O   B
     \/   \/
      \___/          (V)
      /   \
     O     A
      \
       P(=O)y -
      /

     OH
```

in which A, B and y are as previously defined.

When $R^1$ is a labile group removable by acid, the compounds of formula (I) may be regarded as derivatized nucleotides, and will be referred to herein as "monomers"; otherwise, the compounds of formula (I) are polymers or oligomers, and these will be referred to herein, where appropriate in context, as "UDNAP's" (= unnatural DNA probes).

In one embodiment of the invention, the compounds of formula (I) will be derivatized 2′-deoxynucleotides (or correspondingly modified deoxyribonucleic acids or oligonucleotides) which are 2′-substituted by a radical or formula $-NHCO-(CH_2)x-R^3$; the base moiety in such compounds, which will generally be a cytosine, thymidine, adenosine or guanine residue, is unmodified.

In an alternative embodiment of the invention, the compounds of formula (I) will be derivatized 5-substituted-2′-deoxyuridine phosphites or phosphates (or correspondingly modified deoxyribonucleic acids or oligonucleotides), in which the 5-substituent has the formula $-CH{:}CHCH_2-(CH_2)_x-R3$. In this embodiment, x is preferably 3-21.

As regards the compounds of the invention generally, a value for x within the range of from 4 to 8 is particularly preferred.

While it will be apparent that the monomers of the invention may in general be either phosphite or phosphate derivatives, the phosphite derivatives (y = 0) are presently preferred.

The radical $R^1$ may be (besides a hydrogen atom) generally any labile group removable by acids, provided that such group does not interfere with the other relevant reactions.

The monomers of the invention may in general be prepared by a process which comprises reacting with a reagent introducing the labile group $R^1$, the analogue of the desired compound in which $R^1$ is H. Further elaboration of the preparative processes utilized in the present invention will be give hereinbelow.

The monomers of the invention which are derivatized 2′-deoxynucleotides, 2′-substituted by a radical of formula $-NHCO-(CH_2)x-R^3$, may be prepared by a process which comprises reacting with a reactive ester of $HOOC-(CH_2)x-R^3$, the analogue of the desired compound in which A is primary amino instead of $-NHCO-(CH_2)x-R^3$.

The derivatized 5-substituted 2-deoxyuridine phosphite and phosphate monomers of the invention are preferably prepared by first forming a deoxyuridine-5-mercuric compound and then reacting this with a palladium-containing catalyst and a compound of formula $CH_2{=}CHCH_2-(CH_2)x\ -R^3$, where $R^3$ is as defined above above except primary amino, the intermediate of formula (IX)

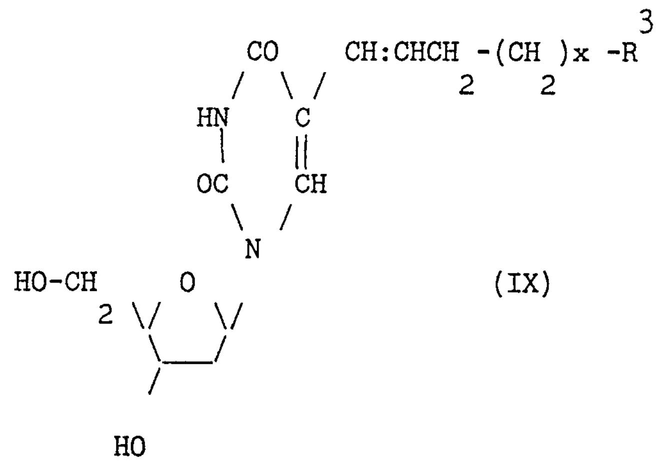

(IX)

then being treated to introduce successively the labile group $R^1$ and the phosphorus-containing group $-(R^2)-P(O\text{-alkyl})(=O)y$.

Further elaboration of the preparative processes utilized for preparing the monomers of the present invention will be given hereinbelow.

In a particular aspect, the present invention also relates to modified deoxyribonucleic acids (or corresponding oligonucleotides) as defined above, whether these substances are derived from derivatized 2′-substituted-2′-deoxynucleotides or from derivatized 5-substituted-2′-deoxyuridine phosphites or phosphates. It is preferred generally that at least two units of formula (V) (above) are present, and that 4 to 20 natural nucleotide units separate consecutive units of formula (V).

These polymers or oligomers may be prepared by reacting the monomers (in which $R^3$ is other than primary amino) with a supported and protected 2′-deoxynucleotide or oligo-2′-deoxynucleotide which has been made by a method known per se. Further elaboration of the preparative processes utilized for preparing the polymers or oligomers of the present invention will be given hereinbelow.

In yet a further aspect, there is provided a method for the detection of a bacterium, fungus, virus, viroid or other source of nucleic acids, comprising the steps of hybridization with at least one polymeric or oligomeric phosphate of formula (I) and detecting the group $R^3$ in the thus-formed hybrid or hybrids, by a non-radioactive method. Further details will be given below.

## DETAILED DESCRIPTION OF THE INVENTION

In one embodiment, the present invention relates to DNA probes incorporating modified nucleotides, i.e. 5-substituted-2′-deoxyuridines or 2′-substituted-2′-deoxynucleotides, the substituent in both cases including a "linker arm" to which is attached a reporter group which enables identification and location of the derivatives to be carried out without radiolabelling. In order for a modified nucleotide to be generally suitable for the task while avoiding radioactive labelling, the following requirements should be satisfied:

(1) the DNA probe must contain a reporter gorup which is unique and not found in natural DNA or RNA;

(2) the reporter group must react specifically with chemical or biological reagents so as to possess a sensitive detection system;

(3) the analogues should not be placed in a position that could interfere with the normal Watson-Crick model, otherwise the reporter groups will decrease the melting temperature that could play an important role in the hybridization of small DNA probes like the UDNAP of this invention;

(4) the reporter groups should be in a position to facilitate accessibility of the chemical or biological reagents in order to enhance the sensitivity and to prevent quenching of the fluorophores during the hybridization when using solid supports like nitrocellulose filters or microtiter plates; and

(5) the "linker arm" should be stable under all experimental conditions to which normal polynucleotides are subjected e.g. during the cycle of the chemical synthesis, deprotection and purification using electrophoresis, solvent (e.g. butanol) extractions and hybridization at elevated temperatures.

All of these requirements are satisfied by the UDNAPs of the present invention described herein.

In the compounds of formula (I), the radical $R^1$ may be (besides a hydrogen atom) generally any labile group removable by acids, provided that such group does not interfere with the other relevant reactions; it is presently preferred that such radical be selected from trityl, monomethoxytrityl, dimethoxytrityl and

trimethoxytrityl. This radical may be introduced at a convenient stage in the preparative process (see below) by utilizing a reagent introducing it, for reaction with the analogue of the desired compound in which $R^1$ is H.

Preferred values for $R^3$ are a biotin residue, dansyl, alkanamido such as acetylamino, trifluoroacetylamino, alkoxycarbonyl, a sugar moiety bound as glycoside such as alpha-D-mannopyranoside, or other moieties capable of detection by ELISA or other non-radioactive means, in particular other moieties capable of forming a detectable complex with a polypeptide when the compound is incorporated in a double-stranded duplex formed with a complementary ribonucleic or deoxyribonucleic acid. Examples of such other moieties are dinitrophenyl, fluorescein, rhodamine, nitroiodophenylacetic acid, a steroid such as oximino-O-derivatized testosteroneoxime, a derivative of luminol or isoluminol, and the residues of any other antigen recognizable by monoclonal or polyclonal antibodies. Reference to a "biotin residue" herein is intended to convey that such residue may be attached to the rest of the molecule in any convenient manner, as for example by an amide link.

Moreover, since aromatic moieties intercalate into the hybrid DNA-DNA or DNA-RNA, it is preferred that the moiety $R^3$ be non-aromatic with a "linker arm" of at least about C5 attached thereto, so sufficient space will be present to permit formation of stable complexes.

It will be appreciated, however, that $R^3$ so far as the monomers per se are concerned, need not be itself a directly detectable radical, but may be a radical which is merely a starting point or an intermediate in the formation of a suitable detectable radical or complex.

The monomers of the invention which are derivatized 2'-deoxynucleotides, 2'-substituted by a radical of formula $-NHCO-(CH_2)x-R^3$, may be prepared by a process which comprises reacting with a reactive ester of $HOOC-(CH_2)x-R^3$, the analogue of the desired compound in which A is primary amino instead of $-NHCO-(CH_2)x-R3$.

The thus-formed NH-CO bond is not cleavable during the synthetic cycle of oligonucleotides including treatment with 2% trichloroacetic acid in dichloromethane, oxidation with iodine/lutidine in water/THF, and deprotection with thiophenol and with concentrated ammonia at 60°C.

Preferably, the derivatized 2'-substituted-2'-deoxynucleotide monomers of the invention where R is Cl are prepared by a process which comprises the following steps, namely:

(i) reacting the compound of formula (VI)

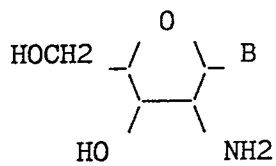

(VI)

with a reactive ester of $HOOC-(CH_2)x-R^3$, or with the free acid itself in presence of a catalyst, to give the product of formula (VII),

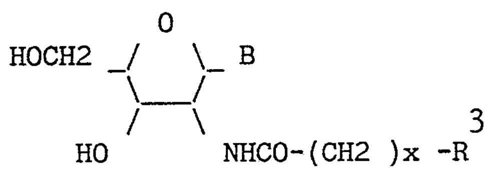

(VII)

(ii) the compound of formula (VII) is reacted with a reagent introducing the labile group $R^1$ to give the compound of formula (VIII)

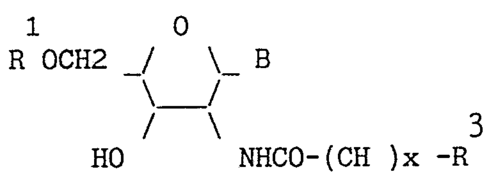

(VIII)

and (iii) the compound of formula (VIII) is reacted with alkyl-O-P(=O)y$Cl_2$ in presence of a tertiary amine.

In the above process, the preparation of the required starting material (VI) may be exemplified, e.g., by describing how 2'-deoxyuridine is obtained. Uridine and diphenyl carbonate in hexamethylphosphoramide are reacted at an elevated temperature such as 150°, for say 20 minutes, in presence of sodium bicarbonate, the product is mixed with water, extracted with chloroform and the aqueous phase evaporated to dryness. The product is reacted with sodium azide in DMF for a prolonged period at elevated temperature, e.g. at 95° for 5 hours and then at 150° for 10 hours, the solvent removed and the residue purified by e.g. chromatographing on silica gel using 4:1 chloroform/methanol as eluent. The azide product is dissolved in dioxan, treated with concentrated ammonia and excess triphenyl phosphine, the mixture stirred one day at room temperature, the solvents evaporated, the residue partitioned between benzene and water, and the aqueous layer was concentrated by evaporation.

Step (i) is then effected on the residue without purification, using DCC (if the required free acid is reacted) in a mixture of 1:1 DMF/methylene chloride, e.g. at room temperature for 4 hours, filtering off the dicyclohexylurea, and the product separated by e.g. chromatographing on silica gel using 4:1 chloroform/methanol as eluent (Rf = 0.45), and further purified by crystallization from chloroform/methanol. In place of the reaction with DCC, a reactive ester such as the p-nitrophenyl ester of the acid may be used, in slight excess e.g. 0.2 mole excess of the stoichiometric requirements, in an 18 hour reaction (for example) at room temperature.

The reactive ester which may be used in step (i) can be, for example, the ester with p-nitrophenol or with N-hydroxysuccinimide. The reactant acid, or the acid from which the reactive ester is derived, may e.g. be biotin, or for example may have the formula, HOOC-$(CH_2)_5$-Q, where Q may be acetamido, trifluoroacetamido or alkoxycarbonyl.

Step (ii) is then effected on the product of step (i) by reacting it with substantially an equimolar amount of (optionally mono-, di-or tri-methoxy-substituted)trityl chloride, or using a slight excess such as 0.2 mole excess of the reagent, such as dimethoxytrityl chloride, in a suitable solvent and in presence of a tertiary base such as pyridine, for about 4 hours at room temperature; excess dry pyridine can also be used as the solvent. Subsequently, the pyridine hydrochloride byproduct is removed, the solvent is evaporated, and the product of this step is preferably purified. The latter step may be effected as e.g. by chromatographing on silica gel, using an aprotic solvent such as chloroform, THF, ethyl acetate, dichloromethane or a solvent mixture, as the eluent. An eluent comprising 5% methanol, 94% chloroform and 1% pyridine is especially suitable.

Step (iii) may then be effected by using e.g. $MeOPCl_2$ in a slight excess, e.g. a 0.2 molar excess, in a THF/collidine mixture at (e.g.) -70°C for 10-30, preferably 20 minutes, and the product is stored under nitrogen at -70°C.

The derivatized 5-substituted 2'-deoxyuridine phosphite and phosphate monomers of the invention are preferably prepared by a process which comprises the following steps, namely:

(a) reacting 2'-deoxyuridine with a reagent mercuric compound to form a deoxyuridine-5-mercuric compound;

(b) reacting the latter with a catalyst comprising a palladium compound and with a compound of formula

$CH_2=CHCH_2-(CH_2)x$ $-R^3$, where $R^3$ is

as defined above except primary amino;

(c) treating the mixture with a sulfide reagent and filtering off the insoluble matter comprising mercuric sulfide;

(d) recovering from the filtrate the intermediate of formula (IX);

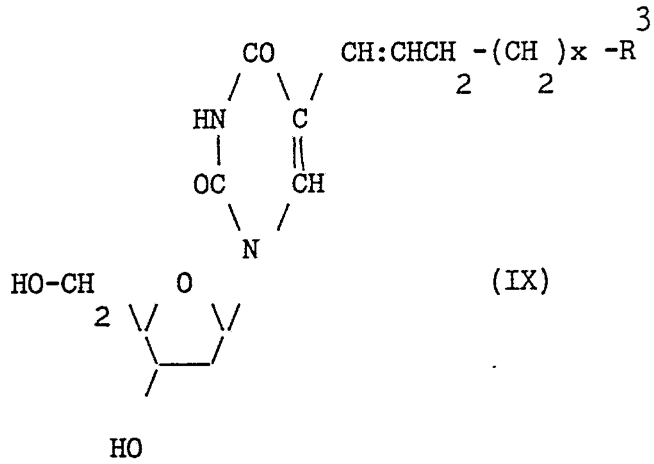

(IX)

(e) reacting compound (IX) with a reagent introducing the labile group $R^1$ in the 5′-position;

(f) reacting the product of the previous step with a reagent of formula alkyl-O-P(X) (=O)y-$R^2$, where X is a leaving group, and optionally carrying out one or both of the following, namely:

(i) where it is desired to obtain the end-product when $R^3$ is primary amino, converting by a method known per se, the product in which $R^3$ is acylamido;

(ii) where it is desired to obtain the end-product when $R^3$ is selected from the group consisting of biotinamido and dansyl, in the case where such compound was not prepared by the process up to step (f), or where it is desired to obtain the end-product when $R^3$ is a different acylamido radical from that prepared by the process up to step (f), reacting the amino-compound of step (i) with a reactant selected from the group consisting of biotinamide, dansyl and an acylating agent which will give the desired different acylamido radical.

In the multi-step process just described, step (a) is conveniently carried out by stirring in aqueous solution at about 50°C for about 4 hours. The reagent mercuric compound may be for example mercuric alkanecarboxylate such as the acetate, or a mercuric halide such as the chloride.

Step (b) is conveniently carried out in a solvent comprising methanol, and is advantageously effected in the presence of an anhydrous cupric compound such as $CuCl_2$. The palladium compound may be for example selected from alkali metal/palladium halides,alkali metal/palladium alkanecarboxylates and triphenylphosphine palladium halides. Suitable such palladium compounds are e.g. lithium or potassium palladium chloride, lithium palladium acetate and triphenylphosphine palladium chloride.

The sulfide reagent in step (c) is preferably used after allowing the reaction mixture to stand for say up to 24 hours; such reagent is suitably hydrogen sulfide or an ammonium or alkali metal sulfides. Recovery step (d) may be effected, for example, by chromatography on silica gel.

Step (e) may be effected by reacting the intermediate (IX) with substantially an equimolar amount of (optionally mono-, di-or tri-methoxy-substituted)trityl chloride, in a suitable solvent and in presence of a tertiary base such as pyridine, for about 4 hours at room temperature; subsequently, the pyridine hydrochloride byproduct is removed, the solvent is evaporated, and the product of this stage is preferably purified, as e.g. by chromatographing on silica gel.

Step (f) may suitably be effected in a reaction medium such as methylene chloride, in the presence of a tertiary base (which acts as a scavenger for HX) such as diisopropylethylamine. It is at present preferred to use in step (f), a reagent of formula alkyl′-O-P(X) (=O)y-NR′R″, where alkyl′ is methyl or ethyl, in order to obtain a product in which $R^2$ is NR′R″ and $R^4$ is methyl or ethyl. A suitable reaction time will generally be up to about 30 minutes at room temperature. Following the reaction in step (f), the desired product may be extracted with a suitable solvent such as ethyl acetate, and the extract is desirably co-evaporated with benzene and the product lyophilized.

With regard to the aspect of the present invention which relates to modified deoxyribonucleic acids (or corresponding oligonucleotides) as defined above, these substances are derived from derivatized 2′-substituted-2′-deoxynucleotide monomers or from derivatized 5-substituted-2′-deoxyuridine monomers, and may be either phosphites or phosphates. It is preferred generally that at least two units of formula (V) (above) are present, and that 4 to 20 natural nucleotide units separate consecutive units of formula (V).

In these polymers or oligomers, $R^3$ may be selected, for example, from a biotin residue, dansyl, alkanamido such as acetylamino, trifluoroacetylamino, alkoxycarbonyl, a sugar moiety bound as glycoside such as alpha-D-mannopyranoside, or other moieties capable of detection by ELISA or other non-radioactive means, in particular other moieties capable of forming a detectable complex with a polypeptide

when the compound is incorporated in a double-stranded duplex formed with a complementary ribonucleic or deoxyribonucleic acid. The other moieties may be e.g. dinitrophenyl, fluorescein, rhodamine, nitroiodophenylacetic acid, a steroid, a derivative of luminol or isoluminol, or the residue of any other antigen recognizable by monoclonal or polyclonal antibodies.

It will be appreciated, however, that even in the polymers or oligimers, $R^3$ need not be itself a directly detectable radical, but may be a radical which is merely a starting point or an intermediate in the formation of a suitable detectable radical or complex.

The polymers or oligomers of the invention have the formula (I) in which $R^1$ is a radical of formula (III) and $R^2$ is a radical of formula (IV), subject to the proviso that formula (I) then represents a modified deoxyribonucleic acid (or a corresponding oligonucleotide) in which 1 to about 100 monomeric units in any position(s) have been replaced by the same number of units of formula (V), above, in which the symbols are as previously defined. That is to say, when z is > 1, the plurality of units of formula (V) present in the compound are not necessarily adjacent to each other; the general formula in this aspect of the invention is merely a shorthand manner of indicating that either one or a plurality of such units may be present in the molecule.

The polymers or oligomers of the invention may be prepared by initially reacting the monomers (in which $R^3$ is other than primary amino) with a supported and protected 2′-deoxynucleotide or oligo-2′-deoxynucleotide which has been synthesized by a method known per se, and if and when desired, any one or more of the following further steps may be carried out, namely:

(i) the conversion of acylamido to amino by a method known per se, and/or

(ii) the reaction of amino formed in step (i) with biotinamide or with dansyl, or with an acylating agent which will give the desired value for $R^3$ where this is acylamido in the product, or with any other compound of structure such that in the product it has become converted to a moiety capable of forming a detectable complex with a polypeptide when the compound is incorporated into a double-stranded duplex formed with a complementary ribonucleic or deoxyribonucleic acid, and/or

(iii) the oxidation of any phosphite ester residues present to phosphate ester residues.

The desired value for $R^3$, which is a moiety capable of forming a detectable complex as described, may be derived from, for example, (in addition to the previously specified biotinamide and dansyl), luminol derivatives, fluorescein, steroids such as testosterone O-(carboxymethyl)oxime (which is detectable by monoclonal antibodies developed against it), 2-nitro-4-iodophenylacetic acid and rhodamine.

The novel UDNAP (i.e. the polymer or oligomer) of the invention may be kinased and ligated normally in order to provide longer UDNAP strands and duplexes. Any desired single UDNAP strand can be synthesized using small complementary segments as templates for ligation.

Thus, the length of polynucleotide (UDNAP) in which m and n in formula (I) represent integers up to about 1000, can be achieved by synthesis from small fragments of UDNAP, of 25-40 units in length, and in parallel synthesis of relatively small polynucleotides that are complementary to the UDNAP. The relatively more elongated compounds can be made from the relatively shorter fragments by using enzymes like T-4 polynucleotide kinase and T-4 DNA ligase. Also, elongation can be effected simultaneously with more than two UDNAPs by ligating in one step three or more UDNAPs with their appropriate templates in a so-called "shot gun" ligation.

Also, DNA duplexes may be synthesized, for example, by synthesizing two or more different, linear DNA strands which are formed into short duplex strands. Each duplex strand preferably includes a double stranded region of from about 20 to about 200 selected complementary base pairs and top and bottom single strand regions. The top single strand includes monomer units of formula (V) (above), preferably separated by 4 to 6 other bases, and the bottom single strand is natural complementary DNA. Each duplex is then annealed to one or two different duplex strands having a complementary single stranded terminal sequence, thereby forming a single continuous double stranded DNA sequence.

According to a preferred embodiment, at least three different duplex DNA strands are prepared and all strands so prepared are annealed concurrently in a single annealing reaction mixture to form a single continuous double stranded DNA sequence.

The present invention thus also provides a duplex strand derived from DNA characterized by the presence of:

(i) a double stranded region of from 10 to about 1000, preferably from about 20 to about 200, complementary base pairs,

(ii) a top single strand comprising a polymeric or oligomeric phosphate of formula (I); and

(iii) a bottom single strand of natural complementary DNA.

It is preferred that in the top single strand, at least two units of formula (V) are present, and any two consecutive such units are separated from each other by about 4 to 20 units, more preferably from 4 to 6

units, of the naturally occurring nucleotides.

One or more of the duplex strands of the invention may be subjected to an annealing reaction.

With regard to the aspect of the present invention relating to the detection of a bacterium, fungus virus, viroid or other source of nucleic acids, which comprises the steps of hybridization with at least one polymeric or oligomeric phosphate of formula (I) and detecting the group $R^3$ in the thus-formed hybrid of hybrids, by a non-radioactive method, this may, for example, be carried out by a procedure comprising the steps of:

(i) hybridization with the a polymeric or oligomeric phosphate of formula (I);

(ii) removing the hybrid from the reaction mixture and complexing with the polypeptide appropriate for the particular $R^3$ group present; and

(iii) characterizing the polypeptide so as to enable identification and estimation of the source of nucleic acids to be effected.

Thus, where $R^3$ is a biotin residue, the polypeptide may be, for example, avidine or streptavidine, to either of which is attached a residue selected from fluorescein, rhodamine, peroxidase and alkaline phosphatase residues.

In this connection, it is pointed out that in general the reporter groups which have been attached either in the monomer, or subsequently in the polymer of oligomer, may be detected by contacting the hybrid UDNAP-DNA or UDNAP-RNA with polypeptides or with indicator molecules which are capable of forming complexes under suitable conditions in order to form one or more moieties which can be detected by conventional techniques. When the reporter group comprises a biotin residue, the polypeptide detector for it may be avidine. If avidine is coupled with indicator molecules such as fluorescein, rhodamine or enzymes (e.g. peroxidase or alkaline phosphatase), then the presence, location and/or quantity of the biotin probe can be detected. But since avidine binds by non-specific interactions with nucleic acids as reported by Heggeness, Stain Technol. 52: 165 (1977) and Bayer and Wilchek, Methods of Biochemical Analysis 26: 1 (1980), a more preferred polypeptide for forming a complex with UDNAP-biotin is streptavidine, which has a much lower non-specific binding to DNA than avidine, see Hoffman et al, Proc. Natl. Acad. Sci. 77: 4666 (1980).

Alternatively, $R^3$ may be for example the residue of a steroid (such as testosterone, progesterone, estradiol or derivatives of any of these) or of any other antigen recognizable by monoclonal or polyclonal antibodies and the polypeptide is comprised by monoclonal or polyclonal antibodies which are characterized by techniques known per se.

The steroids may be rendered antigenic by covalent linkage to bovine serum albumin (BSA) through an appropriate position of the steroid, and by (for example) the O-(carboxymethyl)oxime group. A preferred detectable steroid-containing group is testosterone O-(carboxymethyl)oxime which was used to immunize rats followed by polyethylene glycol-induced hybridization of the immune lymphocytes with mouse myeloma cells so as to form hybridomas that were cloned, and the antibodies produced by each clone were characterized. All of the antibodies obtained showed high affinity for testosterone ($K^a = 10^{10}$/mol). Large quantities of the selected specific antibodies can be obtained by mass growth of the hybridoma line in cultures, or as tumors in irradiated or nude mice.

In yet another alternative, $R^3$ may be for example a dinitrophenyl group or the residue of nitroiodophenylacetic acid.

The detection method of the invention may be applied, for example, to detect a plant virus or viroid. In this instance, a suitable procedure comprises the steps of:

grinding at least one leaf disk of a plant to be detected in presence of an extraction buffer;

centrifuging the extract (e.g. for about 2 minutes);

heating the supernatant to an elevated temperature, e.g. to about 100°C, suitably for about 3 minutes;

spotting the heated supernatant on nitrocellulose paper presoaked in aqueous NaCl and sodium citrate (using e.g. substantially equimolar amounts of these salts) or the chemical equivalents thereof;

pouring a solution of the complementary polymeric or oligomeric phosphate of the invention onto the spotted nitrocellulose paper;

washing with saline solution to remove unhybridized reagent; and

detecting the group $R^3$ in a manner known per se.

The detection method of the invention may be applied, for example, to detect a source of nucleic acids selected from the group consisting of avocado sunblotch viroid, tobacco mosaic virus and citrus exocortis viroid.

In a variation of the detection method of the invention, the target is hybridized with two species of the polymeric or oligomeric phosphate of formula (I), and the thus-formed hybrids are separated and detected. More particularly, it is preferred that in this variation, after first separating the target to be identified or

estimated, it is hybridized with the two species (these being preferably one in which $R^3$ comprises a biotin residue and one in which $R^3$ comprises a steroid residue), the hybrid(s) are separated using e.g. microtiter plates and/or immunobeads and/or sticks coated with monoclonal or polyclonal antibodies against the steroid or other antigen, followed by washing out the unhybridized target and/or unreacted reagent, and finally the hybridized target in which $R^3$ comprises a biotin residue is detected by means of reagents selected from the group consisting of streptavidine-peroxidase, streptavidine-alkaline phosphatase, streptavidine/fluorescein, streptavidine/rhodamine, streptavidine/dansyl and streptavidine/isoluminol derivatives and the enzyme residue is detected more especially by means selected from ELISA, chemiluminescence and fluorescence.

A particularly advantageous manner of practising the detection method of the invention comprises the steps of:

(i) reacting a polymeric or oligomeric phosphate of formula (I) containing a suitably reactive terminal group $R^3$ with a polypeptide containing a plurality of reactive radicals, selected from free $NH^2$ and $CO^2H$ radicals, so as to form covalent bonding between said phosphate and the polypeptide (the polypeptide being conveniently first coated onto microtiter plates, beads or sticks);

(ii) removing unreacted said phosphate;

(iii) hybridizing consecutively or simultaneously the target with the polypeptide reaction product thus prepared and with a separately prepared polymeric or oligomeric phosphate of formula (I) containing a detectable $R^3$ ($R^{3'}$) radical;

(iv) removing unreacted components; and

(v) detecting the radical $R^{3'}$ in the doubly-hybridized target.

At least one of the two removing steps (ii) and (iv) is preferably effected by washing.

In this last-mentioned manner of practising the detection method of the invention, the detectable $R^3$, radical is preferably a biotin residue, and in stage (v) this residue may be detected in the doubly-hybridized target by means of <u>either</u> [A] streptavidine-enzyme or avidine-enzyme, whereby the enzyme (which may for example be peroxidase or alkaline phosphatase) is detected by means of a method selected from the group consisting of ELISA, chemiluminescence and fluorescence; <u>or</u> [B] streptavidine/dye or avidine/dye, and especially a dye selected from fluorescein, rhodamine, dansyl and isoluminol derivatives.

In this manner of practising the detection method of the invention, the detectable $R^{3'}$ radical need not necessarily be a biotin residue; it may otherwise be for example, a radical selected from dansyl, nitrophenyl, the residue of fluorescein, rhodamine, nitroiodophenylacetic acid, or of a steroid, a derivative of luminol or isoluminol, or the residue of any other antigen recognizable by monoclonal or polyclonal antibodies, and in stage (v) such a detectable radical is detected by a method known <u>per se.</u>

This manner of practising the detection method of the invention, which involves covalent bonding between the $R^3$ group of the monomeric unit (V), possesses the following advantages, namely:

(1) the polypeptide used in the initial stage may be e.g. bovine serum albumin (BSA), which is relatively cheap, compared with possible alternatives such as monoclonals or polyclonals;

(2) the formation of covalent bonding in accordance with the invention, between the UDNAP and the polypeptide, leads to much more stable entities, e.g. by comparison, the antigen/antibody bond in a possible alternative operation has a dissociation constant of $K = 10^{-7}$, and this antigen/antibody bond is liable to be cleaved in the hybridization process, which is not the case with a covalent bond; and

(3) the testing procedure is very rapid, since after 10 minutes, a detection signal is obtained.

In one embodiment of the present invention the reporter group is coupled to the monomer before the synthesis of the DNA probe. This has proved possible because the reporter group, $R^3$, does not interfere in the annealing procedure or during enzymatic work such as kination or ligation. Even biotin when used as a reporter group is not oxidized by $I^2/H^2O$/lutidine during DNA synthesis as might have been anticipated.

According to an alternative embodiment, the DNA probes which are first synthesized incorporate monomeric units of formula (V) according to he present invention, which have a side chains terminating in a suitably reactive group, and the desired reporter group is then attached thereto.

Both of these embodiments permit insertion of the monomeric units (V) in the desired locations and at the desired spacing along the length of the DNA probe, which is not possible with most prior art methods.

The UDNAP's of the present invention may be used in biomedical research, chemotherapeutic agents, clinical diagnosis and recombinant DNA technology. These various utilities are based at least in part upon the ability of the molecules to form stable complexes with polypeptides which can in turn be detected, either by means of properties inherent in the polypeptides, or by means of detectable moieties which are attached to, or interact with, the polypeptides. Some more specific potential uses include detecting and identifying nucleic acid-containing etiological agents, e.g. bacteria and viruses (e.g. in herpes or cytomegalovirus chlamidia), screening bacteria for antibiotic resistance, diagnosing genetic disorders such

as thalassemia and stickle cell anemia, chromosomal karotyping, and identifying tumor cells.

As regards the detection method of the invention generally, it will be appreciated that the hybridization step may be effected in solution, followed by fishing out the hybrid or hybrids. The versatility of this method is shown by embodiments using more than one UDNAP reporter groups. The method of hybridization in solution increases the degree of hybridization and enhances sensitivity. The saving of time during the hybridization in solution, as against the method of utilizing solid support, renders this technique a powerful new instrument for clinical diagnostics - diagnosis may be performed in a very short time, e.g. in several minutes or up to one hour, without using radio isotopes.

The invention is illustrated by the following non-limiting Examples.

EXAMPLE I

2,2′-anhydro-1-(beta-D-arabinofuranosyl)uracil.

This procedure is according to Moffatt et al, J. Org. Chem. 36: 250 (1971).

Uridine (38 g.) and diphenyl carbonate (44.4 g.) were dissolved in hexamethylphosphoramide (150 ml.), sodium bicarbonate (1.0 g.) was added, and the mixture was heated at 150°C for 20 minutes. TLC (1:1 ethyl acetate/methanol, $R_f$ = 0.55) showed that essentially only the title product was present. The mixture was cooled, added to water (1.21.) and extracted 3 x with chloroform. The aqueous phase was evaporated to dryness and the residue crystallized from methanol giving 31.0g. (88%) of pure product, m.p. 238-240°C.

EXAMPLE II

2′-azido-2′-deoxyuridine.

A magnetically-stirred mixture of 3.1 g. (10 mmol.) of the preceding product and 3.26 g. (50 mmol.) of sodium azide in dry DMF (100 ml.) was heated for 5 hours at 95°C and then for 10 hours at 150°C. Removal of the DMF under reduced pressure and chromatography of the residue on a 3.0 x 40 cm. column of silica gel using flash chromatography, eluting with 4:1 chloroform/methanol (v/v) gave 3.41 g. (96.8%). $R_f$ = 0.47.

NMR (DMSO-d) 3.62 (m, 2H), 3.89 (m, 1H), 4.03 (t, 1H, J = 5.5 Hz), 4.31 (t, 1H, J = 5.5 Hz), 5.67 (d, 1H, J = 8 Hz), 5.90 (d, 1H, J = 5.5 Hz), 7.90 (d, 1H, J = 8 Hz).

Calcd. for $C_9H_{11}N_5O_5$: C, 40.15; H, 4.12; N, 26.0. Found: C, 39.81; H, 4.43; N, 26.10.

EXAMPLE III

2′-amino-2′-deoxyuridine.

The procedure used was according to Moffatt et al (loc cit). A solution of 2′-azido-2′deoxyuridine (1.34 g., 5 mmol.) in methanol (20 ml.) was vigorously stirred in a hydrogen atmosphere with 10% Pd/C catalyst (700 mg.) for 40 minutes. The mixture was filtered and evaporated, leaving 1.18 g. (98%) of crystalline product, m.p. 192-3°. After twice recrystallizing from ethanol, it had m.p. 197-8°C.

EXAMPLE IV

2′-biotinamido-2′-deoxyuridine.

A solution of 2′-amino-2′deoxyuridine (2.43 g., 10 mmol.), biotin (3.42 g., 14 mmol.) and dicyclohexylcarbodiimide (2.88 g., 14 mmol.) in a 1:1 mixture of dichloromethane/DMF (50 ml.) was mixed overnight at room temperature. Dicyclohexylurea was filtered off, the filtrate was evaporated to dryness. The residue was dissolved in 4:1 (v/v) chloroform/methanol (15 ml.) and chromatographed on a column of 150 g. silical gel using flash chromatography, the crude product being eluted with the same solvent mixture. $R_f$ = 0.14. The product was a white solid, m.p. 212-4°C (dec.). yield 4.09 g. (87%).

NMR (pyridine-d5) 2.18-2.07 (m, 6H), 3.97 (g, 1H, J = 12 Hz), 4.12 (m, 5H), 4.50 (bvs, 1H), 4.86 (bvd, 1H, 5.5 Hz), 5.32 (bvh, 1H, J = 8 Hz), 5.73 (d, 1H, J = 8 Hz), 6.83 (d, 1H, J = 8 Hz), 8.37 (d, 1H, J = 8 Hz), 9.60 (bv, 3H).

Calcd. for $C_{19}H_{27}N_5O_7S$: C, 48.61; H, 5.75; N, 14.92; S, 6.82. Found: C, 48.47; H, 5.80; N, 14.97; S, 6.77.

EXAMPLE V

2′-biotinamido-5′-dimethoxytrityl-2′-deoxyuridine.

2′-biotinamido-2′-deoxyuridine (4.70 g., 10 mmol.) was dissolved in anhydrous pyridine (100 ml.) and the solution was evaporated to dryness. The resultant solid was dissolved in pyridine (20 ml.) and 4,4′-dimethoxytrityl chloride (4.06 g., 12 mmol.) was added. The mixture was shaken in the dark for 5 hours. (Silica gel TLC of the product was conducted in 4:1 (v/v) chloroform/methanol containing 1% pyridine. $R_f$ = 0.35.) Methanol (5 ml.) was added, the mixture evaporated to a gum and the residue was extracted with ethyl acetate (200 ml.). The organic phase was washed with water (50 ml.) and brine (100 ml.) and evaporated to dryness.

The crude product was separated by flash chromatography on silica gel (150 g.), using a gradient of solvents from 95:4:1 to 80:19:1 chloroform/methanol/pyridine. The product was evaporated to dryness and lyophilized with benzene to give a white powder, m.p. , yield 5.91 g. (77.5%).

NMR (deutero-chloroform) 2.18-2.07 (m, 6H), 3.74 ( , 6H), 3.97 (m, 1H), 4.14 (m, 5H), 4.55 (br, S, 1H), 4.86 (bvd, 1H, J = 5.5 Hz), 5.38 (m, 1H), 5.73 (d, 1H, J = 8 Hz), 6.83 (d, 1H, J = 8 Hz), 7.30 (d, 13H, J = 8 Hz), 8.37 (d, 1H, J = 8 Hz), 8.60 (m, 3H).

EXAMPLE VI

6-(trifluoroacetamido)hexanoic acid.

Trifluoroacetic anhydride (40 ml., 270 mmol.) was slowly added to 6-aminohexanoic acid (13.1 g., 100 mmol.). The rate of addition was controlled so that insufficient heat was generated to evaporate off the anhydride. After all of the anhydride had been added, the homogenous solution was kept for 1 hour at room temperature, and evaporated to a thick syrup which was mixed with cold water (100 ml.) and stirred overnight. The crystalline product was filtered off, washed with cold water, dried and recrystallized from ether. M.p. 88-9°C, yield: 61%.

EXAMPLE VII

2′-[6-(trifluoroacetamido)hexanamido]-2′-deoxyuridine.

2′-amino-2′-deoxyuridine (2.43 g., 10 mmol.) and 6-(trifluoroacetamido)hexanoic acid 4.54 g., 20 mmol.) were dissolved in a mixture of 1:1 dichloromethane/DMF (50 ml.), and dicyclohexylcarbodiimide (2.88 g., 14 mmol.) was added. The solution was stirred overnight at room temperature, and dicyclohexylurea was filtered off. The mixture was evaporated to dryness and the product was dissolved in 4:1 (v/v) chloroform/methanol (10 ml.), chromatographed on a column of silica gel (150 g.) using flash chromatography, and the crude was eluted using a gradient of solvents from 96: 4 to 80:20 chloroform/methanol. $R_f$ = 0.39. The product was a glassy oil, yield 3.88 g. (85.8%).

NMR (pyridine-d5) 1.31 (S, 6H), 2.14 (m, 2H), 3.33 (br, t, 2H, J = 8 Hz), 4.07 (d, 2H, J = 12 Hz), 4.50 (br, S, 1H), 4.86 (br, d, 1H, J = 5.5 Hz), 5.32 (br, H, 1H, J = 8 Hz), 5.73 (d, 1H, J = 8 Hz), 6.83 (d, 1H, J = 8 Hz), 8.37 (d, 1H, J = 8 Hz).

EXAMPLE VII

2'-[6-(trifluoroacetamido)hexanamido]-5'-dimethoxytrityl-2'-deoxyuridine .

2'-[6-(trifluoroacetamido)hexanamido]-2'-deoxyuridine (4.42 g., 10 mmol.) was dissolved in anhydrous pyridine (100 ml.) and the solution was evaporated to dryness. The resultant solid was dissolved in pyridine (20 ml.) and 4,4'-dimethoxytrityl chloride (4.06 g., 12 mmol.) was added. The mixture was shaken in the dark for 5 hours. (Silica gel TLC of the product was conducted in 98:2 (v/v) ethyl acetate/methanol. $R_f$ = 0.39.) Methanol (5 ml.) was added, the mixture evaporated to a gum and the residue was extracted with ethyl acetate (200 ml.). The organic phase was washed with water (50 ml.) and brine (100 ml.), dried over sodium sulfate and evaporated to dryness.

The crude product was separated by flash chromatography on silica gel (200 g.), eluting with 98:2 (v/v) ethyl acetate/methanol. The product was evaporated to dryness and lyophilized with benzene to give a white powder, m.p. , yield 6.98 g. (95%).

NMR (deutero-chloroform) 1.31 (S, 6H), 2.17 (m, 2H), 3.42 (br, t, 2H, J = 8 Hz), 3.73 (S, 6H), 4.12 (S, 3H), 4.75 (br, d, 1H, J = 5.5 Hz), 5.35 (br, h, 1H, J = 8Hz), 5.74 (d, 1H, J = 8 Hz), 6.85 (d, 1H, J = 8 Hz), 7.35 (br, 13H), 7.85 (d, 1H, J = 8 Hz).

EXAMPLE IX

Hydrolysis of 2'-[6-a(trifluoroacetamido)hexanamido]-2'-deoxyuridine. (THD)

A solution of THD (4.52 g., 10 mmol.) and potassium carbonate (4.2 g., 13 mmol.), methanol (20 ml.) and water (80 ml.) was stirred at room temperature under nitrogen for 20 hours. The end of the reaction was monitored by TLC with 4:1 chloroform/methanol. $R_f$ = 0.1. After neutralization with 5N HCl to pH 7.0, the mixture was evaporated to dryness and the residue was dissolved in a mixture of 1:1 ethyl acetate/methanol. The mixture was filtered and the solvents were evaporated to leave a residual oil.

EXAMPLE X

Testosterone-3-[(O-carboxymethyl)oxime]-17-acetate.

Testosterone (3.8 g., 13 mmol.) and O-(carboxymethyl)hydroxylamine hemihydrochloride (4.0 g., 18 mmol) were refluxed for 90 minutes in ethanol (750 ml.) containing 75 ml. N NaOH solution. The reaction mixture was evaporated under reduced pressure to a volume of 100 ml., diluted with water and extracted with ether. The alkaline aqueous phase was acidified with concentrated hydrochloric acid to pH 4.5. The resulting precipitate was extracted with ether, the ether extract washed with water, dried over sodium sulfate and evaporated to dryness. Three recrystallizations from benzene-hexane yielded 3.25 g. (68%) of product, m.p. 179-81 °C (dec.).

$a_D^{25}$ = 143.8 +/- 2° (19.5 mg. in 1.5 ml. of ethanol).

UV (methanol): max 252nm (~21,000).

NMR (DMSO-d6) 0.67 (S, methyl-18), 1.02 (S, methyl-19), 4.50 (S, $CH_2$-oximino), 5.72 (S, H4).

The oxime (1 g.) was acetylated by dissolving in anhydrous pyridine (30 ml.) and acetic anhydride (20 ml.) at room temperature, and allowing the mixture to stand overnight. The solvents were evaporated off and the crude residue was mixed with water (50 ml.) at room temperature for 5 hours.

EXAMPLE XI

Phosphomonochloridites of the products of Examples V and VIII.

The procedure of Letsinger et al J. Am. Chem. Soc. 98:3655-3661 (1976) was used. Either product (1 mmol.) was dissolved in a mixture of tetrahydrofuran (5 ml.) and collidine (0.5 ml.). The solution was stirred at -78 °C under nitrogen, then methyl phosphorodichloridite (75 ul., 0.8 mmol.) was added. The solution was stirred for 20 minutes and stored under nitrogen at -70 °C. Each phosphomonochloridite solution was used in oligonucleotide synthesis without any purification.

EXAMPLE XII

Synthesis of modified DNA Probe (UDNAP)

The preparation of deoxy-oligonucleotides, including 2'deoxyuridine substituted at the 2'-position, was carried out according to the procedure of Letsinger et al J. Am. Chem. Soc. 98:3655-3661 (1976) and of Caruthers et al, Tetr. Letters, 21:719-722 (1980) and J. Am. Chem. Soc. 103:3185-3191 (1981). The synthesis is commenced by derivatizing controlled pore glass (CPG; Pierce Co.) (500 Å ) that attaches via alkylamine and covalently bonded dicarboxylic acids with a terminal free carboxylic acid function to the 3'-hydroxyl group of protected (e.g. 5'-dimethoxytritylated) nucleosides.

The chemical steps used for the addition of one nucleotide to this support are as follows:

(1) detritylation of the supported nucleoside using 2% trichloroacetic acid in dichloromethane (30 seconds);

(2) condensation of the appropriate 5'-dimethoxytrityl-2'-deoxynucleotide-3'-(methyl phosphomonochloridite) with the thus-produced supported deprotected nucleoside over a 10 minute period, using a solution of 50 mg. of the latter dissolved in 0.5 ml. THF + 5 equivalents of collidine relative to the chloridite;

(3) blocking unreacted support-bound nucleoside hydroxyl groups with 1:1 acetic anhydride/2,6-lutidine (2 minutes);

(4) oxidation of phosphite to phosphate with 0.01M in 1:0.5 2,6-lutidine/0.5 THF (2 minutes)

This procedure may be repeated as desired to give a deoxyoligonucleotide. After the final condensation step, including removal of the terminal dimethoxytrityl group, the deoxyoligonucleotide is purified and isolated as follows. The CPG including deoxyoligonucleotides is washed thoroughly with methanol and air-dried. The esterifing methyl group on the phosphate esters is removed by treating the polymer (100 mg. containing 2-5 micromols. deoxyoligonucleotide) with 2 ml. of a 1:2:2 thiophenol/triethylamine/dioxan mixture for 75 minutes at room temperature. This step is followed by treatment with concentrated ammonia solution for 2 hours at 20°C, to hydrolyze the carboxylic ester groups by which the deoxyoligonucleotides are joined to the support.

After centrifugation and recovery of the supernatant containing the deoxyoligonucleotides, the base protection groups are removed by warming in a sealed tube at 50°C for 24 hours. The ammoniacal solution is then evaporated to dryness. The residue is redissolved in 2 ml. of water, filtered, and the solution is washed three times with n-butanol (3 x 2 ml.). Final purification of the product is by electrophoresis on 20% polyacrylamide gels using a tris-borate buffer (pH 8) containing 7M urea. The crude material [approximately 10 O.D. units (260 nm) in 30 ul. of 80% formamide] is loaded into a well 2.5 cm. wide and electrophoresis is conducted at 16v./cm. To detect DNA bands, the gel is placed on a fluorescent TLC plate (Sigma Chemical Co.) and illuminated with long UV light in order to visualize the deoxyoligonucleotides which appear as intensely absorbing bands in each lane. The desired band is cut from the gel and the DNA is eluted with 0.5M ammonium acetate, 10 nM magnesium chloride, 0.1% SDS and 0.1 nM EDTA. After two washings with n-butanol, the deoxyoligonucleotides are desalted on a Sephadex G50/40 column (45 x 2.5 cm.) using 10 nM TEAB (pH 7.0). Recoveries from 10 O.D. units of crude DNA generally range from 1.0 to 2.0 O.D. units (260 nM).

EXAMPLE XIII

Preparation of specific modified DNA probes.

This example specifically illustrates the preparation of unnatural DNA probes (UDNAPs) for use in hybridization in relation to:

(A) Avocado Sunblotch Viroid;

(B) Citrus Exocortis Viroid; and

(C) Tobacco Mosaic Virus.

Eight segments of UDNAP were synthesized, two for Viroid (A), three for Viroid (B) and two for Virus (C). Three short complementary segments were synthesized which then served as a template for ligation procedures, as shown from the following scheme:

1a - - - - - - - - - - 2a - - - - - - - - - - -

K - - - - - - - -

All segments were synthesized according to the general procedure of Example XII.

Three segments constructed for use to detect Avocado Sunblotch Viroid had the following sequences:

1a) 3' TTUGGTCCAGACAAGGCU GAAA from 46-67

2a) GGCUGAGACUCAAAGCUGAA from 68-87

3a) CACUCTCTU CCTCCUCAGCACCA from 88-106

K) TCAGCCTTTCAG complementary to 1a and 2a

Two segments constructed for use to detect Citrus Exocortis Viroid had the following sequences:

1b) 3' GGCCCCUTTGGACCUCCTU from 95-113

2b) CAGCUCCAGCCCCCCC from 114-129

M) GAGCTGAAGGAG complementary to 1b and 2b

Two segments constructed for use to detect Tobacco Mosaic Virus had the following sequences:

1c) TCCACA UGTCCAUGTUA from 5800-5817

2c) CGCCAUAATCUGGGCGAUCA from 5818-5837

N) TATGGCGTAACA complementary to 1c and 2c

The overall yield of the synthesis was 20% from the intensity of the first condensation.

In the following Examples XIV-XVI, "UDNAP-A" [see also Example XIX, part (c) et seq] represents for example a deoxypoly-or deoxyoligo-nucleotide in which the 2'-deoxyuridine phosphate residues have been replaced by analogous 2'-substituted-2'-deoxyuridine residues, the 2'-substituent being (e.g.) $-NHCO-(CH_2)_5-NH_2$, in which the terminal $-NH_2$ is produced in the polymer or oligomer by a procedure analogous to that described in Example IX above, i.e. by room temperature hydrolysis of the N-trifluoroacetyl analogue.

In Example XVII, "UDNAP-3a" is the oligonucleotide described in Example XIII, where each U is a 2'-substituted-2'-deoxyuridine residue, the 2'-substituent being $-NHCO-(CH_2)_5-NH_2$, in which the terminal $-NH_2$ is produced in the polymer or oligomer by a procedure analogous to that described in Example IX above, i.e. by room temperature hydrolysis of the N-trifluoroacetyl analogue.

## EXAMPLE XIV

Coupling of UDNAP-A with Fluorescein Isothiocyanate.

Fluorescein isothiocyanate (1 mg.) was dissolved in dry DMF (50 ul.). To this solution was added a solution of UDNAP-A (0.5 O.D.) in 0.1 M sodium bicarbonate (100 ul.). The solution was shaken for 30 minutes at room temperature and then loaded onto a Sephadex G-50 column (10 ml.) and eluted with double distilled water. After 2 ml. of void volume, 5ml. of the eluent was collected and lyophilized.

## EXAMPLE XV

Coupling of UDNAP-A with 6-N-(4-isothiocyanatobutyl)-N-ethylamino-2,3-dihydrophthalazine-1,4-dione (ABEI-ITC)

ABEI-ITC (1 mg.) was dissolved in dry DMF (50 ul.). To this solution was added a solution of UDNAP-A (0.5 O.D.) in 0.1 M sodium bicarbonate (100 ul.). The solution was shaken for 30 minutes at room temperature and then loaded onto a Sephadex G-50 column (10 ml.) and eluted with double distilled water. After 2 ml. of void volume, 5 ml. of the eluent was collected and lyophilized.

EXAMPLE XVI

Coupling of UDNAP-A with N-hydroxysuccinimidobiotin.

N-hydroxysuccinimidobiotin (1 mg., Sigma) was dissolved in dry DMF (50 ul.). To this solution was added a solution of UDNAP-A (0.5 O.D.) in 0.1 M sodium bicarbonate (100 ul.). The solution was shaken for 30 minutes at room temperature and then loaded onto a Sephadex G-50 column (10 ml.) and eluted with double distilled water. After 2 ml. of void volume, 5 ml. of the eluent was collected and lyophilized.

EXAMPLE XVII

Coupling of UDNAP-3a with Testosterone-3-(O-carboxymethyl)oxime.

Testosterone-3-(O-carboxymethyl)oxime (10 mg.) and N-hydroxysuccinimide (5 mg.) were placed in an Eppendorf and dissolved in dry dichloromethane (1 ml.). To this solution was added dicyclohexylcarbodiimide (8.4 mg.) The solution was stirred at 4°C overnight and then centrifuged in order to remove the dicyclohexylurea. 100 ul. of this solution was evaporated to dryness and redissolved in 10 ul. of dry DMF. To this solution was added a solution of UDNAP-3a (1-O.D.) in 100 ul. of 0.13 M sodium bicarbonate. The solution was shaken for 30 minutes, loaded onto a Sephadex G-50 fine column (10 ml.) and eluted with double distilled water. UV absorption analysis of the antigen-UDNAP complex indicated that it contained three residues derivatized testosterone per mole of UDNAP-3a.

In Examples XVIII and XIX, unless the context otherwise dictates, the term "testosterone" will be used as a shorthand expression for testosterone in which the keto group O= is replaced by a substituted nitrogen -N= (in the form of an oxime ether), as described herein.

EXAMPLE XVIII

Preparation of Monoclonal Antibodies for Testosterone

This example describes the establishment of hybridoma lines that produce monoclonal antibodies to testosterone, and the use of these antibodies in the development of a solid phase in conjunction with liquid phase ELISA for the measurement of the markers that are attached to UDNAP so as to utilize a double hybridization technique. This is a novel application of monoclonal antibodies as reagents in immunotrapping for steroid hormones in order to fish out the DNA or RNA target to be tested by DNA-DNA or DNA-RNA hybrid that could be detected by means of ELISA, fluorescence or chemiluminescence.

The hybridoma lines were derived from fusion of mouse myeloma cells with spleen cells of rats immunized with testosterone-3-(O-carboxymethyl)-oxime bovine serum albumin conjugate as described by Lindner et al, Steroids, 39: 453-9, 1982.

Synthesis of testosterone-3-(O-carboxymethyl)-oxime bovine serum albumin conjugate.

Testosterone 3-(O-N-hydroxysuccinimidomethyl)-oxime (10 mg.) in dimethylformamide (0.5 ml.) was added to a solution of bovine serum albumin (20 mg.) in 0.13M $NaHCO_3$ (0.5 ml.). The mixture was stirred for 15 mins. and dialyzed against 0.1M $NaHCO_3$ and distilled water. The product was freeze-dried. Ultraviolet absorption analysis of the antigenic complex indicated that the immunogen contained 16 steroid residues per mole of BSA.

Immunization: Wistar-derived female rats (2 to 3 months old) from the Departmental Colony were immunized by an intradermal injection of testosterone-3-(O-carboxymethyl)-oxime bovine serum albumin conjugate (200 ug/rat) incorporated in complete Freund's adjuvant. A month later the injection was repeated, and booster injections (100 ug/rat) were given three weeks later. All animals were bled from the tail ten days after the booster injection, and the antisera were tested then for titer and specificity.

Cell lines and culture conditions: The non-Ig producer mouse myeloma cell line NSO/1 used in the fusion experiments was kindly supplied by Dr. Z. Eshhar, The Weizmann Institute, Israel. Cells were cultured at 37°C in a humid atmosphere of 10% $CO_2$ in the air. Dulbecco's modified Eagle's medium (DMEM) (high glucose, Gibco, N.Y.), supplemented with glutamine (2 mM), streptomycin (100 ug/ml) and

penicillin (100 units/ml) was used throughout. Selective growth medium (HAT) contained hypoxanthine (0.1mM), aminopterin (0.4 uM) and thymidine (0.16 uM) in Dulbecco's modified medium with 15% heat inactivated horse serum.

Cell hybridization: On days 4 and 3 before the fusion experiment, rats were given a booster injection (100 ug/rat) in saline. Spleen cells were then removed and fused with the mouse myeloma cells by using 41% polyethyleneglycol (Serva, Type 1550, Heidelberg, West Germany). After fusion the cells were distributed in Costar tissue culture plates (Cluster 24, Costar, Cambridge, Mass.), each well containing less than 5 x $10^4$ cells/ml. The hybrids arising in each well were selected in Dulbecco's modified Eagle's medium (DMEM) containing hypoxanthine, aminopterin and thymidine (HAT).

Cloning of cells: Hybrid cells that secreted antibodies to testosterone-3-BSA were cloned by limiting dilutions of 96-well microplates in Dulbecco's modified Eagle's medium (DMEM) containing hypoxanthine and thymidine (HT). Subcloning of stable and high secreting clones were performed in soft agar medium with spleen cells as feeders.

Growth of hybridomas in mice: Two weeks prior to cell inoculation, adult male mice (BALB/c x DBA/z) $F_1$ or BALB/c nude mice received an intraperitoneal (i.p.) injection of 2,6,10,14-tetramethylpentadecane (Pristane, Aldrich Chemicals, Wisconsin) (0.5 ml/mouse). The $F_1$ mice were in addition radiated with $^{60}$Co at 750 rad for 10 mins.; cultured hybridoma cells (2-3 x $10^7$) in 5 ml Dulbecco's modified Eagle's medium were subsequently injected i.p. into the irradiated or nude mice. Ascitic fluid (5-15 ml/mouse) was harvested 10 to 20 days after hybridoma inoculation.

Characterization of antibodies : A dextran-coated charcoal radioimmunoassay method was used to detect specific antibodies to testosterone. Antibody titer denotes the dilution at which 50% of the labelled ligand, added at concentrations of 50 pg./ml., is bound as determined by radioimmunoassay. Cross reactivity (%) was calculated as proposed by Thorneycroft et al ("Immunologic Methods", Steroid Determination, Peron and Caldwell, eds., Appleton-Century-Crofts, New York 1970, pp. 63-86). as 100x/y where x is the mass of testosterone and y is the mass of heterologous compound required to produce 50% inhibition of the binding of the tritiated ligand. Affinity of the antibodies was calculated according to Abraham et al in Handbook of Radioimmunoassay, Marcel Dekker Inc., New York, 1977, pp. 591-656.

Preparation of monoclonal antibodies to testosterone: Fusion of spleen cells of immunized rats with the mouse myeloma cells resulted in the growth of hybrid cells in 26% of the culture wells (147/566). After two weeks of growth, antibody-secreting hybridomas were detected by a RIA procedure in the tissue culture media of 30 out of the 147 cultures. During subsequent cell culture, 3 hybridoma lines that yielded high antibody titers were cloned by limiting dilutions and sub-cloned on semi-solid agar. When injected i.p. into pristane-treated irradiated $F_1$ mice or nude mice, the hybridomas grew as tumors and secreted high amounts of anti-testosterone antibodies into the ascitic fluid of these mice, with titers ranging from 1:10,000 to 1:60,000 (Table 1).

Characterization of monoclonal antibodies: The ascitic fluid of mice derived from three hybridomas. $H_6$, $F_2$ and $D_{11}$ was further characterized for affinity and specificity by a dextran-coated RIA procedure. The results are shown in Table 2.

Table 1: Properties of various antibodies to testosterone.

| | Monoclonal Antibodies (Clone #) | | | Polyclonal* Rat IgG |
|---|---|---|---|---|
| | $H_6^X$ | $D_{11}$ | $F_2^X$ | |
| Titer[a] | 1:60,000 | 1:50,000 | 1:10,000 | 1:5,000 |
| Ka (l/mol)[b] | $2.1 \times 10^{10}$ | $1.85 \times 10^{10}$ | $1 \times 10^{10}$ | $3.7 \times 10^{9}$ |
| Heavy chain class[c] | $Y_{2a}$ | $Y_{2a}$ | IgM | |

[a] Titer as defined in the Methods section.
[b] Calculated according to the method of Abraham et al
[c] Determined by double immunodiffusion in agar.
[*] The polyclonal IgG was from the rats and was used for the fusion experiments.

Table 2: Specificity of various antibodies to testosterone as determined by RIA.

| Compound | Cross-reaction (%) Monoclonal $H_6$ | $D_{11}$ | $F_2$ | Polyclonal Rat IgG |
|---|---|---|---|---|
| Testosterone | 100 | 100 | 100 | 100 |
| 5-alpha-Dihydrotestosterone | 100 | 100 | 2 | 10 |
| 5-beta-Dihydrotestosterone | 0.5 | 8.5 | 15 | 2 |

EXAMPLE XIX

Detection of Avocado Sunblotch Viroid (ASBV) by double hybridization technique.

(a) Avocado sunblotch is a serious disease affecting the performance and fruit quality of avocado ( Persea americana). A low molecular weight circular RNA has been associated with the disease and infectivity studies with highly purified RNA preparations have recently confirmed its viroid etiology. The sequence of 247 nucleotide residues of the single-stranded circular RNA from avocado sun blotch viroid was determined and a secondary structure model, in which 67% of the ASBV residues are paired, was proposed.

Sunblotch diagnosis using graft transmission to indicator seedlings is laborious and time-consuming as 1 1/2 - 2 years may be required for characteristic symptoms to develop. Recently, several rapid diagnostic methods based on electrophoretic fractionation of RNA from infected plants in polyacrylamide gels (see Palukaitis, P., Hatta, T., Alexander D-McE and Symons, R.H., "Characterization of a viroid associated with avocado sunblotch disease", Virology 99 : 145-151 (1979)), based on liquid hybridization with an ASBV complementary DNA (cDNA) probe, and based on dot spot self hybridization of $^{32}$P-ASBV-RNA probe and the ASBV-RNA molecules spotted on nitrocellulose paper have been developed for ASBV detection.

However, an alternative technique is described herein using a double hybridization technique which includes in situ double hybridization in solution while the detection of the hybrid is done on a solid support.

(b) Polyvinyl chloride microtiter plate was coated with monoclonal antibodies for testosterone (25 ug/ml) which was diluted in PBS. 120 ul from this solution was added per well, and was left for three hours at 37°C (unbound monoclonal antibodies were collected and reused). The wells were blocked with 150 ul of blocking buffer containing 0.05% Tween 20 and 5 mg/BSA in PBS, for two hours at 37°C. Then washings with the blocking buffer were effected three times, and twice with PBS to which 0.16M NaCl was added, and later the microtiter plates were washed extensively with double distilled water.

(c) UDNAP-3a-Testosterone, as prepared in Example XVII, (0.1 O.D.) which is complementary to ASBV RNA bases 88-110,

```
       Tes  Tes   Tes
3'      |    |     |      5'
    CACUCTCTUCCTCCUCAGCACCA
```

(where on each U there is a testosterone moiety, containing in place of O=, *-N= substituted with a linker arm of 11 atoms [constituted by (2')-NHCO-$(CH_2)_5$-NH-$COCH_2O$-*] attached by its other end to the 2'-position of the 2'-uridine phosphate unit) is lyophilized to dryness. (In an alternative procedure using 5-substituted instead of 2'-substituted 2'-deoxyuridine phosphate units, the linker arm is constituted by (5)-CH=CH-$(CH_2)$-r-NH-$COCH_2O$-*, where r is 8 or 9; such 5-substituted compounds are prepared by analogy with the 2'-substituted compounds, but using appropriate 5-substituted starting materials, see the later Examples.)

UDNAP-A-Biotin (see Example XVI; or alternatively the similarly prepared 5-substituted analogues may be used) which is complementary to ASBV-RNA bases 46-87 with a sequence

TTUGGTCCAGACAAGGCUGAAAGGCUGAGACUCAAAGCUGAA

was synthesized by using fully protected deoxynucleotide. This segment was synthesized by joining UDNAP-1a and UDNAP-2a when K segment served as a template.

(d) Procedure for labelling deoxyoligonucleotides.

In order to get one long segment comprising the synthesized portions joined to one another, it is necessary to label the 5' of the oligonucleotides with ($\gamma$-$^{32}$P)-ATP. The UDNAP 1a and 2a (1 nmole) were each dissolved separately (34 ul) in 50 nM of HEPES (pH 7.6), 10 mM $MgCl_2$ , 10 mM DTT and ($\gamma$-$^{32}$P)-ATP [2 nmole, specific activity = (2 - $^{2}$5) x $10^3$ cpm/mole] and incubated with 1 unit of T4-polynucleotide kinase for 40 mins. at 37°C. The reaction mixture was warmed to 70°C for 5 mins. to inactivate the enzyme. For ligation reactions, the phosphorylated deoxyoligonucleotides were used directly without further purification. In the following discussion, 5'-$^{32}$P denotes a phosphate radioactively labelled at the 5' end of the deoxyoligonucleotides. For monitoring the yield of kination, 1 ul of 1a and 2a were loaded on DEAE-81 paper and eluted with 0.35 M ammonium formate.

(e) Enzymatic joining of segments 1a and 2a in order to get UDNAP A.

1a, [5'-32P]2a and K (500 pmole each) were lyophilized to dryness in a 1.5 ml Eppendorf tube. The deoxyoligonucleotides were dissolved in 30 ul of 50 mM HEPES (pH 7.6), 10 mM $MgCl_2$, and 10 mM DTT. In a second tube,[5'-$^{32}$P]2a (500 pmole) was dissolved in 30 ul of the same buffer. The two mixtures were combined and the volume adjusted to 100 ul with the above buffer. The solution was next warmed in a

boiling water bath for 3 mins., slowly cooled to room temperature, and cooled to 4°C. The concentration of ATP was adjusted to 150 mM. After the addition of 2 ul of 1.0 M DTT and 2 units of T4-DNA ligase, the mixture was incubated for 18 hours at 4°C. The reaction was monitored by electrophoresis on 12% polyacrylamide gel using a tris-borate buffer (pH 8.0) containing 7M urea. After 18 hours, the reaction was considered complete and the reaction solution was purified by two washings with n-butanol, the UDNAP was desalted on a Sephadex G-50 column (10 x 1 cm) using 10 mM TEAB (pH 7.0). The overall enzymatic operations yield was 75%.

NOTE: The segments 1a and 2a joined to yield A; 1b and 2b to yield B; and 1c and 2c to yield C.

(f) 1-2 g. of Avocado leaf strips were ground in a pestle and mortar in the presence of 4% para-aminosalicylic acid and 1 x STE [10mM Tris-HCl, (pH 8.0), 100 mM NaCl; 1 mM EDTA (pH 8.0)]. This was transferred into a tube containing 8 ml of STE, saturated phenol and 0.1% 8-hydroxyquinoline sulphate, shaken and centrifuged for 10 mins. at 8000 rpm in a Sorval SS 34 rotor.

The upper phase was collected and added to a solution containing 1/9 volume of 3M sodium acetate pH 5.5 and 2 volumes of ethyl alcohol. This solution was kept overnight at -20°C. After centrifuging for 15 mins. at 8000 rpm, the solid was resuspended in 0.4 ml. $H_2O$ or in a hybridization buffer containing 6 x SET (1 x SET = 0.15M NaCl, 0.015M Tris-HCl pH 7.5, 1mM EDTA). 100 ul from this solution was added first to an Eppendorf containing lyophilized UDNAP-A-Biotin (0.1 O.D.), kept at 37°C for 10 mins. and then it was transferred to another Eppendorf containing lyophilized UDNAP-3a-Testosterone (0.1 O.D.). The combined solutions were heated to 100°C for 3 mins. and then allowed to cool slowly to room temperature.

(g) The PVC microtiter wells that were coated with anti-testosterone monoclonals were prehybridized by incubating the wells with a solution containing 2 ml. of 5 x Denhardt's solution [10 x (0.5 g. Ficoll, 0.5 g. polyvinylpyrrolidone, 0.5 g. BSA to 500 ml. water)], 5 x SSPE (2 x SSPE = 17.4 g. NaCl + 2.76 g. sodium dihydrogen phosphate monohydrate and 0.74 g. EDTA pH 7.4, to 1 liter) and 100 ug/ml denatured salmon sperm DNA) at 37°C for 30 mins.

The wells were washed three times with washing solution containing 3 x SSC, rinsed with PBS and incubated at 37°C with 200 ul solution containing the double hybrid UDNAP-A-(Biotin)-ASBV-UDNAP-3a-(testosterone) for 1-6 hours. This was followed by extensive washings with PBS and later on the wells were treated with streptavidin-peroxidase conjugate (Sigma) at 37°C for 20 mins., and then the wells were rinsed with PBS three times.

The wells were then incubated with developer solution containing 6 mg. of imidazol in 8 ml. water, 6 mg. of orthodianisidine in 2 ml. EtOH and 10 ul of 30% hydrogen peroxide, for 5 minutes at 37°C. A brown precipitate that developed was found to have stained the wells after washing with PBS.

## EXAMPLE XX

Preparation of 9-Decenyltrifluoroacetamide

A solution of decenyl chloride (33 g., 189 mmol) in dichloromethane (300 ml) containing tetrabutylammonium Iodide 250 mg is cooled in an ice bath. Sodium azide (13.12g., 201 mmol), dissolved in water (50 ml) is added and the reaction mixture is stirred vigorously at 0°C for 2 hrs. The organic phase is separated, washed with water (2 x 50 ml), and reacted with $MgSO_4$ for 20 hrs. Continued evolution of $N_2$ as very small bubbles is observed during this period.

Trifluoroacetic acid (17.2 ml, 0.22 mmol) is added dropwise to the filtered solution which is thereafter refluxed for 6 hrs. The cooled reaction mixture is washed with saturated aqueous $NaHCO_3$ solution (2 x 50 ml), dried with $MgSO_4$, filtered, and evaporated. The product is distilled in a vacuum oil pump. The yield is 86%.

The characteristics of the product are as follows:

Boiling Point: 108-110°C at 0.1 mm Hg.

H.N.M.R.-($CDCl_3$) =6.5 (m,1H); 5.55-6.05 (m,1H); 5.03 (d,2H, J=2Hz); 4.85 (m,1H); 3.33 (quartet,2H); 2.03 (m,2H); 1.3 (m,12H).

EXAMPLE XXI

Preparation of 5-(9-Decenyltrifluoroacetamide)-2'Deoxyuridine

A solution of (2.28 g, 10 mmol) deoxyuridine and $Hg(oAc)_2$ (3.19 g., 10 mmol) in 30 ml $H_2O$ was heated with stirring at 60°C for 5 hrs.

After removal of acetic acid formed in the reaction and water in vacuo, the white solid residue was suspended in 200 ml THF, and to this solution (7.53 g., 30 mmol) of decenyl trifluoroacetamide and 20 ml of 0.1M $Li_2Pd\ Cl_4$ in MeOH and (0.8 g., 0.5 mmol) of anhydrous CuCl2 was added.

After reflux for 18 hours, the brown suspension was treated with $H_2S$, filtered, and chromatographed on a column of 150 g. silica gel using flash chromatography, and the crude was eluted with ethyl acetate. Rf = 0.5.

The product was obtained as oil at a yield of 58%.

N.M.R. ($CDCl_3$+MeOD): 8.24 (m,1H); 7.63 (s,1H); 6.56 (t,1H,J=6.5Hz); 6.4 (broad m,1H); 6.24 (d,1H,J=15Hz); 4.69 (m,1H); 4.20 (m,1H); 3.98 (narrow m, 2H); 2.38 (m,2H); 2.18 (m,4H); 1.31 (m,12H).
I.R. - Neat - 3490, 3390, 3220, 3100, 1690, 1674, 1660, 1480, 1380, 1280, 1090, 1030, 978 $cm^{-1}$.

EXAMPLE XXII

Preparation of [5-(9-Decenyltrifluoroacetamide)]-5'-Dimethoxytrityl-2'-Deoxyuridine

5 -(9-decenyltrifluoroacetamide)-2'-deoxy uridine (2.65 g., 5.5 mmol) was dissolved in anhydrous pyridine (100 ml) and the solution evaporated to dryness. The resultant solid was dissolved in pyridine (10 ml) and 4,4'-dimethoxytritylchloride (2.0 g., 6 mmol) was added. The mixture was shaken in the dark for 4 hours, whereupon some crystals had separated. Silica gel TLC of the product was conducted in ($2CH_2Cl_2$:1 EtOAc:0.1 pyridine Rf = 0.4). Methanol (4ml) was added, the mixture evaporated to a gum and extracted with ethyl acetate (100 ml) and cold 1M $NaHCO_3$ (40 ml). The organic phase was washed with water (40 ml) and brine (40 ml) and evaporated to dryness in the presence of pyridine.

The crude product was separated by flash chromatography on silica gel 150 g., using a gradient of solvents from 1% pyridine: 99% $CH_2Cl_2$ through 50% $CH_2Cl_2$:49% EtOAc:1% pyridine. The product was evaporated to dryness and lyophilized with benzene to yield a white powder.

The yield of product was 3.06 g. (71%). The product had a melting point of 140°C (decomposed) and N.M.R. spectrum ($CDCl_3$) - 8.60 (broad m,3H); 7.30 (d,13H,J=8Hz); 6.84-6.74 (broad,5H); 6.37 (m,1H); 6.24 d,1H,J=15Hz); 4.49 (m,1H); 4.06 (m,2H); 3.76 (s,6H); 2.38 (m,2H); 2.31 (m,12H).
Elemental analysis:
$C_{42}H_{48}N_3O_8F_3$ Calc. C 64.69 H 6.16 N 5.39
779 Found C 64.60 H 6.17 N 5.36

EXAMPLE XXIII

Preparation of Phosphoramidite of [5-(9-Decenyltrifluoroacetamide)]-5'-dimethoxytrityl-2'-deoxyuridine

5 -(9-Decenyltrifluoroacetamide)-5'-dimethoxytrityl-2'-deoxyuridine (2.34 g., 3 mmol) is placed with a magnetic stirring bar in a (100 ml) round bottom flask subsequently fitted with a serum cap which is wired in place to prevent its loss due to pressure build up. The contents are dried in a vacuum desiccator overnight. The flask is then filled with dry argon, 30 ml of dry $CH_2Cl_2$ is introduced via syringe using an extra needle to vent excess pressure and the mixture is stirred until completely dissolved. The flask is then placed in a cold water bath and 1.8 equivalents of diisopropylethylamine is added by syringe. While the reaction mixture is stirred, 1.2 equivalents of dimethylaminochloromethoxy phosphine (0.4 ml) is slowly added by syringe, at such a rate that the dichloromethane solution does not boil (using a syringe needle to vent excess pressure). After addition of the phosphine is complete, the vent needle is removed and the reaction is stirred for an additional 15 minutes.

The reaction flask is uncapped and the mixture poured into a one liter separatory funnel containing (100 ml) of saturated NaCl solution. The reaction flask is rinsed with 200 ml of ethyl acetate and the rinses added to the separatory funnel. The lower aqueous layer is discarded. The ethyl acetate layer is washed (3 x 100 ml) with saturated NaCl solution. The organic layer is then dried on Anhydrous $Na_2SO_4$ and evaporated to

dryness.

The white foamy residue is then taken up in 50 ml of dry benzene and transferred to a 250 ml round bottom flask. An additional 25 ml of benzene is used to rinse any residue from the large flask into the smaller one. The benzene solution is frozen and then placed on a vacuum line fitted with a large cold trap to collect the benzene as it sublimes. The product is lyophilized overnight. The white powdered phosphoramidite is transferred to a pre-weighed dry wide mouth bottle and stored under argon in the freezer.

N.M.R. ($CDCl_3$) (phosphorous): -147.5 to 146.4 ppm ($CDCl_3$) in respect to 5% aqueous $H_3PO_4$.
Yield - 2.30 g. (95%).

EXAMPLE XXIV

Preparation of Penta-O-acetyl-alpha-D-mannopyranose

D-mannose (12 g., 60 mmole) is added in small portions during the course of half an hour to a mixture of acetic anhydride (100 ml) and pyridine (130 ml), cooled in an ice-salt mixture. The reaction mixture is stirred vigorously for 4 hours with continued cooling and kept 2 days at 0°C with occasional stirring. The solution is poured slowly with vigorous stirring into water (1.5 liters) containing crushed ice. A syrup separates which, after further stirring, crystallizes. The crystals are filtered and washed well with water. Yield 21 g. (79%). The melting point of the product is 117°C. The product is sufficiently pure for use in the next stage, i.e. Example XXV, after thorough drying.

EXAMPLE XXV

Preparation of (10-Undecenyl-2,3,4,6-tetra-O-acetyl-alpha-D-mannopyranoside

Alpha-D-mannopyranoside penta-acetate (3.90 g., 10 mmole) was dried under high vacuum overnight, then dissolved in 10 ml of dry $CH_2Cl_2$. This solution was treated with (2.6g., 10 mmole) of $SnCl_4$ with stirring under argon for 10 minutes. The solution turned violet. Undecenyl alcohol (2.6 g., 15 mmole) in 5 ml $CH_2Cl_2$ was added and the solution was stirred for 4 hours at room temperature. The reaction mixture was then quenched into 5% aqueous $NaHCO_3$.

The mixture was extracted with $CH_2Cl_2$ (2 x 100 ml), washed with water, NaCl solution and dried on $Na_2SO_4$, filtered and concentrated in vacuo to a pale-yellow oil. The product was purified by chromatography on silica gel by elution with 30:70 ethyl acetate/hexane. Rf = 0.6. The product was obtained as a oil, yield 3.34 g.(63%), and has the following characteristics:

$^1$HNMR ($CDCl_3$): 4.40-370 (m, 5H, with d at 4.18, J = 2HZ) 4.79 (m, 2H), 5.50 - 6.04 (m, 1H), 5.24 - 5.40 (m, 2H), 2.1 H (S, H), 2.09 (S, 3H), 2.03 (S, 3H), 1.98 (S, 3H) 1.31 (broad S, 14 H).

Elemental Analysis:
$C_{25}H_{40}O_{10}$ Calc. C 60.00 H 8.00
500 Found C 59.91 H 8.03

EXAMPLE XXVI

Preparation of 5-[10-Undecenyl-(2,3,4,6-tetra-O-acetyl-alpha-D-Mannopyranoside]-2'-Deoxyuridine

A solution of 2'-deoxy uridine (228 mg., 1 mmole) and mercuric acetate (319 mg, 1 mmol) in 10 ml $H_2O$ was heated with stirring at 60°C for 5 hours. A white solid precipitated.

After removal of HOAc formed in the reaction and water in vacuo, the white solid residue was suspended in 50 ml of THF, and with (1.5 g., 3 mmole) of (10-Undecenyl-2,3,4,6-tetra-O-acetyl-alpha-D-Mannopyranoside and with 11 ml of 0.1 M $Li_2PdCl_4$ in MeOH.

The mixture was refluxed for 18 hours, then treated with $H_2S$, filtered and chromatographed on a column of 150 g. of silica gel by flash chromatography using ethyl acetate as eluent. Rf = 0.4. The dried product was a white solid. Recrystallization from EtOAc-Hexane.

$^1$HNMR ($CDCl_3$) - - 8.24 (m,1H); 7.35 (m,1H); 6.78 (m,1H); 6.05 (broad m,1H); 5.24-5.38 (s,3H); 4.80 (sharp

m,1H); 4.30-2.84 (m,12H); 2.15 (s,3H); 2.10 (s,3H); 2.04 (s,3H); 1.99 (s,3H); 1.29 (broad,14H).
Elemental Analysis:
$C_{34}H_{50}N_2O_{15}$ calc. C 56.19 H 6.88 N 3.85
726 found C 56.02 H 6.91 N 3.81

EXAMPLE XXVII

Preparation of [5-(10-Undecenyl-2,3,4,6-tetra-O-acetyl-alpha-D-mannopyranoside)]-5'-dimethoxytrityl-2'-deoxyuridine

5-[10-Undecenyl-(2,3,4,6-tetra-O-acetyl-alpha-D-mannopyranoside]-2'-deoxyuridine (10g., 20 mmole) was dissolved in anhydrous pyridine (100 ml) and the solution evaporated to dryness. The resultant solid was dissolved in pyridine (40 ml), and 4,4'-dimethoxytritylchloride (7.4 g., 22 mmole) was added. The mixture was shaken in the dark for 4 hours in a sealed flask, whereupon some crystals had separated. Silica gel TLC of the product in 15% ethanol/chloroform showed one trityl and uv-positive spot at higher Rf than the starting material. Methanol (4 ml) was added, the mixture evaporated to a gum and partitioned between chloroform (200 ml) and cold 1 M $NaHCO_3$ (80 ml). The organic phase was washed with water (40 ml) and evaporated to dryness in the presence of pyridine. After co-evaporation with toluene, the product was dissolved in dichloromethane/0.1% pyridine and chromatographed on silica gel 60H (400 g, 13 cm diameter) using 500 ml of dichloromethane/0.1% pyridine and 3 L of 5% ethanol/dichloromethane/0.1% pyridine. Pure fractions were pooled, evaporated to a foam with pyridine, dissolved in chloroform (80 ml) and precipitated with pentane (3 l.). The yield was 13.0 g. (82%) of product.

EXAMPLE XXVIII

Synthesis of modified DNA probe

Preparation of deoxyoligonucleotides is carried out according to the general methodologies published in Matteucci, et al., J. Am. Chem. Soc., 103, pp 3185 - 3192 (1981) and Beaucage, et al., Tetrahedron Letters, 22, pp. 1859 - 1862 (1981) and the references cited therein. The synthesis begins by derivatizing high performance liquid chromatography grade silica gel to contain appropriately protected nucleotides. The deoxyoligonucleotides are linked through the 3'-hydroxyl group to a carboxylic acid functional group attached covalently to the silica gel. The chemical steps used for the addition of one nucleotide to this support are as follows: (1) detritylation using $ZnBr_2$ in nitromethane/methanol (4 min.); (2) condensation of a 5'-di-p-anisylphenylmethyl deoxynucleoside 3'-methoxy-N, N-dimethylaminophosphine with the support bound nucleoside (5 min.); (3) blocking unreacted support bound nucleoside hydroxyl groups with acetic anhydride (5 min.); and (4) oxidation of the phosphite to the phosphate with $I_2$ (2 min.). Syntheses are performed in simple sintered glass funnels by a single technician. The time required for one synthetic cycle is 20 to 30 minutes and deoxyoligonucleotides containing up to 30 mononucleotides may be obtained in high yields in less than 15 hours.

Purification and isolation of the deoxyoligonucleotide is completed using the following procedure. After the final condensation step, including removal of the terminal di-p-anisylphenylmethyl group with acid, the silica gels containing the deoxyoligonucleotides are washed thoroughly with methanol and air dried. To remove the methyl group from the phosphotriesers, each polymer (100 mg, containing 2 - 5 micromoles of oligonucleotide) is treated with 2 ml of thiophenol: $Et_3N$: dioxane (1:2:2) solution for 75 min. at room temperature. This step is followed by treatment with concentrated ammonium hydroxide at 20°C for 2 hours to hydrolyze the ester joining the deoxyoligonucleotides to the support.

After centrifugation and recovery of the supernatant containing the deoxyoligonucleotides, the base protection groups are removed by warming in a sealed tube at 50°C for 24 hours. The ammonium hydroxide solution is then evaporated to dryness. The residue is redissolved in 2 ml of $H_2O$, filtered and the solution then washed three times with n-butanol (3 x 2 ml). Final purification of the product is by electrophoresis on 20% polyacrylamide gels using a tris-borate buffer (pH 8) containing 7 M urea. Approximately 10 O.D. units (260 nm) of the crude material in 30 uls of 80% formamide is loaded into a well 2.5 cm wide and electrophoresis is conducted at 16 v/cm. To detect DNA bands, the gel is placed on a fluorescent TLC plate (Sigma Chemical Company) and illuminated with long UV light in order to visualize the deoxyoligonucleotides which appear as intensely absorbing bands in each lane. The desired band is cut

from the gel and the DNA eluted with 0.5 M ammonium acetate, 10 mM $MgCl_2$, 0.1% SDS, and 0.1 mM EDTA. After two washings with n-butanol, the deoxyoligonucleotides are desalted on a Sephadex G50/40 column (45 x 2.5 cm) using 10 mM TEAB (pH 7.0). Recoveries from 10 O.D. units of crude DNA generally range from 1.0 to 2.0 O.D. units (260 nm).

EXAMPLE XXIX

Preparation of specific modified DNA probes

The following example specifically illustrates the preparation of unnatural DNA probes (UDNAP) for use in hybridization to:

A) Avocado sunblotch viroid.
B) Citrus Exocortis viroid.
C) Tobacco Mosaic virus.

Eight segments of UDNAP were synthesized, three for Viroid (A), three for Viroid (B) and two for Virus (C). Five short complementary segments were synthesized which then served as a template for ligation procedures, as shown from the following scheme:

```
        1A                   2A                   3A
  ---------------    ----------------    ---------------
            K ----------          L -----------
```

All segments were synthesized according to the general procedure of Example XXVIII.

Three segments constructed for use to detect Avocado Sunblotch Viroid (ASBV) had the following sequences:

1a) 3′ TTUGGTCCAGACAAGGC UGAAA from 46-67
2a)GGCUGAGACU CAAAGCUGAA from 68-87
3a) CACUCTCT UCCTCCUCAGC from 88-106
K) TCAGCCTTTCAG complementary to 1a and 2a.
L) GAGTGTTCAG complementary to 2a and 3a.

Two segments constructed for use to detect Citrus Exocortis Viroid had the following sequences:

1b) 3′ GGCCCCUTTGGACCUCCTU from 95-113
2b) CAGC UCCAGCCCCCCC from 114-129
M) GAGCTGAAGGAG complementary to 1b and 2b.

Two segments constructed for use to detect Tobacco Mosaic Virus had the following sequences:

1c) TCCACAUGTCCAUGTUA from 5800-5817
2c) CGCCAUAATCUGGGCGAUCA from 5818-5837
N) TATGGCGTAACA

The overall yield of the synthesis was 20% from the intensity of the first condensation.

A. Procedure for Labelling Deoxyoligonucleotides.

In order to get one long segment comprising the synthesized portions joined to one another, it is necessary to label the 5′ of the oligonucleotides with [ -$^{32}$P]-ATP.

The deoxyoligonucleotides (1 nmole) were dissolved (34 ul) in 50 mM of HEPES (pH 7.6), 10 mM $MgCl_2$, 10 mM DTT and [ -$^{32}$P]-ATP (2 nmole, specific activity = (2 - 5) x $10^3$ cpm/pmole) and incubated with 1 unit of T4-polynucleotide kinase for 40 min. at 37°C. The reaction mixture was warmed to 70°C for 5 min. to inactivate the enzyme. For ligation reactions, the phosphorylated deoxyoligonucleotides were used directly without further purification. In the following discussion, [5′-$^{32}$P] denotes a phosphate radioactively labeled at the 5′ end of the deoxyoligonucleotides.

B. Enzymatic Joining of Segments 1a, 2a and 3a in order to get UDNAP A.

1a, [5'-$^{32}$P]2a and K (500 pmole each) were lyophilized to dryness in a 1.5 ml Eppendorf tube. The deoxyoligonucleotides were dissolved in 30 ul of 50 mM HEPES (pH 7.6), 10 mM $MgCl_2$, and 10 mM DTT. In a second tube, [5'-$^{32}$P]3a and L (500 pmole each) were dissolved in 30 ul of the same buffer. The two tubes were warmed in a boiling water bath for 1 minute and slowly cooled to room temperature. The two mixtures were combined and the volume adjusted to 100 ul with the above buffer. The solution was next warmed at 45°C for 2 min and cooled to 4°C. The concentration of ATP was adjusted to 150 mM. After the addition of 2 ul of 1.0 M DTT and 5 units of T4-DNA ligase, the mixture was incubated for 18 hours at 4°C. The reaction was monitored by electrophoresis on 12% polyacrylamide gel using a tris-borate buffer (pH 8.0) containing 7M urea. After 18 hours, the reaction was considered complete and the reaction solution was purified by two washings with n-butanol, the UDNAP was desalted on a Sephadex G-50 column (10 x 1 cm) using 10 mM TEAB (pH 7.0). The overall enzymatic operations yield was 75%.

NOTE: The segments 1a, 2a and 3a, jointed to yield A; 1b and 2b to yield B; and 1c and 2c to yield C.

EXAMPLE XXX

Detection of Avocado sunblotch viroid by hybridization with A

Five leaf disks 1.2 cm in diameter (about 0.15 g.) from healthy and infected plants were ground in a pestle and mortar in the presence of sand and 1 ml of Owens & Diener extraction buffer. The extracts were transferred to 1.5 ml Eppendorf tubes, centrifuged for 2 min and the supernatants were heated for 100°C for 3 minutes. 3 ul samples were spotted on nitrocellulose paper that was presoaked in 20 x SSC.

Oligonucleotide A, which is complementary to ASBV RNA bases 46-106, was synthesized by using fully protected deoxynucleotide. In order to permit secondary testing to confirm the incorporation of the modified monomer and the hybridization of oligonucleotide A as a UDNAP probe, radioactive labelling is provided. For $^{32}$P-labelling of the oligonucleotides, 1 nM from the fragment was dissolved in 35 ul of 50 mM HEPES, 10 mM $MgCl_2$, 10 mM DTT (pH 7.6) containing 2-3 nM of $^{32}$P-ATP, specific activity 3000 pm/nM, and incubated with 1 unit of T4-polynucleotide kinase (Boehringen) for 40 min at 37°C. The reaction was then warmed to 70°C for 5 min to activate the enzyme, and the mixture of free nucleotides and labelled digonucleotides was loaded on a 5 cm Sephadex G-50 column, eluted with water, and fractions containing labelled oligonucleotides were pooled and mixed with 8 ml of hybridization solution containing 3 ml of formamide 2 ml of SSC x 20, 2 ml of Denhardt's solution, 0.5 ml of a 5% DNA salmon sperm solution and 0.5 ml of 1 M phosphate buffer pH 6.8.

In order to confirm the specificity of hybridization of the synthesized DNA probes, leaf extracts from healthy and citrus exocortis viroid (CEV) infected etrog Citrus Medica and Gynura aurantiaca plants and from healthy and ASBV infected avocado plants were spotted on nitrocellulose paper and hybridized with $^{32}$P-labelled synthetic probe "A" discussed above, which is specific to ASBV. Only the ASBV infected samples, (Section A, spots 12-15), gave position hybridization.

Sap samples from ASBV infected plants were serially diluted and found to give positive hybridization at sample dilutions of 1:64-256 (Section B), thus indicating the sensitivity of the DNA probe.

The results of dot spot hybridization of plant extracts from five healthy and eleven ASBV infected plants, including a symptomless ASBV isolate were as follows (Section C). All infected plants gave positive hybridization in at least one of the sampled leaves. However only 13 out of 20 sampled leaves gave clear hybridization whereas 5 of the sampled gave very weak hybridization and two of the infected samples and all five healthy samples were negative.

Separation of ASBV or other viroid infected propagation material from healthy ones at present is mainly based on bioassay on indicator plants and PAGE electrophoresis. These assays are slow and laborious (see Spiegel, et al, Pl. Mol. Biol. 2, 15 - 18 (1983)). Hybridization with ASBV RNA to samples that have been attached to a solid support in accordance with the present invention are 16-64 times more sensitive than PAGE. The higher sensitivity of hybridization appears to be extremely important for NSRN diagnosis because previous studies showed large variations in the viroid concentration in various portions of the infected plants, ranging from 0.1 to 2 x $10^{-5}$ by RNA weight. Dilution experiments carried out in the present study clearly indicate that, even within the leaves of the same plant, 100 times variation of viroid content are quite common.

EXAMPLE XXXI

Reaction of UDNAP A with Nitroiodophenylacetic succinic anhydride (NIPSuc.)

1 nmole of A was lyophilized to dryness and dissolved in 1 ml of $NaHCO_3$ Buffer, pH 9.2. To this solution, a suspension of 2 mg. of NIPSuc in 0.5 ml DMF was added and shaken vigorously for 20 min at room temperature. After washing with -BuOH, the resulting 150 ul of the yellow solution was chromatographed on Sephadex G-50 column (10 x 1 cm) and eluted with water. The product was eluted after 8 ml of void volume.

The product was yellow and has a visible absorption 7 max 430 and comprises a DNA strand having NIP substituted onto the side chains of modified deoxyuridine monomers incorporated along the length thereof.

EXAMPLE XXXII

Preparation of 5-(9-Decenylamine)-5'-dimethoxytrityl-2'-deoxyuridine

A mixture of 5-(9-decenyltrifluoroacetamide)-5'-dimethoxytrityl-2'-deoxyuridine (14.022 g., 18 mmole), potassium carbonate (4.2 g., 30 mmol), methanol (20 ml) and water (80 ml) is stirred at room temperature under nitrogen for 20 hours. The end of the reaction is monitored by TLC with 10 MeOH: 90 EtOAc. After concentration by means of a rotary evaporator, the reaction mixture is extracted with ethyl acetate (3 x 100 ml). The dried $Na_2SO_4$ organic phase is evaporated to dryness and the residue used for condensation with biotin without any purification. (See Example XXXIII).

EXAMPLE XXXIII

Preparation of [5-(9-Decenylamine) biotin]-5'-dimethoxytrityl-2'-deoxyuridine

(2.44 g., 10 mmol) of biotin dissolved in 50 ml DMF was added to a solution of 5-(9-decenylamine)-5'-dimethoxytrityl-2'-deoxyuridine (6.83g., 10 mmol) and 2.26g., 11 mmol) dicyclohexylcarbodiimide (DCC) in 15 ml DMF. The mixture was stirred at room temperature for 12 hours. Dicyclohexylurea (DCU) was filtered out and the product was extracted (3 x 250 ml) with EtOAc. The organic phase was dried on $Na_2SO_4$ and evaporated to dryness.

The yellow solid was chromatographed on silica gel by flash chromatography with 5 MeOH: 95 $CHCl_3$ as eluent. Rf: 0.50. Yield : 49%.

Melting point : 160 - 162° (decomposed)

N.M.R. ($CDCL_3$) - 8.57 (m,narrow 6H); 7.60 (M,6H); 7.30(d,13H,J=8Hz); 6.84-6.74 (broad,5H); 6.35 (m,1H); 6.25 (m,3H); 4.2-4.6 (m,6H); 4.12 (m,4H); 3.74 (s,6H); 3.36-2.85 (m,12H); 2.18-2.07 (m,6H); 1.69-1.20 (broad,18H).

Elemental analysis:

$C_{50}H_{63}N_5O_9S$ calc. C 66.00 H 6.93 N 7.70 S 3.52

found C 65.32 H 6.99 N 7.61 S 3.59

EXAMPLE XXXIV

Detection of Citrus Exocortis Viroid (CEV) by a Double Hybridization Technique

This method involves the formation of a covalent linkage between UDNAP, and a polypeptide coated onto a polyvinyl chloride or polystyrene microtiter plate.

(a) Synthesis of two UDNAP's for CEV.

Two UDNAP (deoxyoligonucleotide) segments which are complementary to CEV at 67-106 and 244-284 were synthesized.

The first segment has the following sequence:

3' CCGCCCCCUTCTTCAGGAAGTCCCTAGGGGCCCCUTTGGA 5' (1)

where each U contains at C-5 a linker arm of 11 atoms terminated by amino [$-CH:CH(CH_2)_8CH_2NH_2$].

The second segment has the following sequence:
3′ CGGGCCUCGAAGAGAGACCTATGAUGGGCCACCTATGTUGA 5′ (2)
where each U contains at C-5 a linker arm of 11 atoms terminated by biotinamido [as above, but the $NH_2$ is replaced by biotinamido].

(b) Coating the plates.
A microtiter plate (Limbro) was coated overnight at 37°C with a solution containing 10% BSA in phosphate buffer saline (PBS) at pH 7.6; thereafter the plate was rinsed repeatedly with double distilled water.

(c) Covalent bonding of UDNAP to coated plates.
Lyophilized segment (1) (1-O.D.) was mixed with a solution of 1-ethyl-3-(3-dimethylaminopropyl)-carbodiimide.HCl (10 mg.) (Sigma) in 1200 ul. water at pH 4.5. 200 ul. portions of this solution were added to six wells which had previously been coated with BSA. After incubation at 37°C for 5 hours, the excess (unreacted) solutions were recovered from the wells and stored at -20°C for re-use. The wells were rinsed twice with double distilled water.

(d) Double hybridization.
Lyophilized segment (2) (0.1 O.D.) was dissolved in a solution of 1400 ul. (6 x SSC, 10 x Denhardt, 0.5% SDS) containing a CEV cloned in PBR 322 (1 ug.). This solution was boiled for 5 minutes and allowed to cool slowly to room temperature. 200 ul. portions of this solution were added to seven wells, of which six contained segment (1) covalently linked to BSA; the seventh well was coated only with BSA and served as a control. The wells were kept at 37°C for 15 minutes and the excess (unhybridized) content of the wells was recovered, and stored at -20°C for re-use. The seven wells were washed 5 x at room temperature with a solution containing (6 x SSC + 0.1% SDS), 5 minutes for each washing. A similar washing process was effected at 50°C, 2 x 15 minutes each, and finally using as a single wash (2 x SSC + 0.1% SDS) at 37°C for 15 minutes.

(e) Detection of the target.
Following the washing, the wells were incubated at room temperature for 10 minutes with a solution of streptavidine-alkaline phosphatase (Bio-Yeda), 10 ug./ml. in STMT buffer (1M NaCl, 0.1M tris-HCl pH 7.5, 2mM $MgCl_2$, 0.05% Triton X-100) and the washed with STMT buffer (3 x 5 minutes each) and STM buffer (1M NaCl, 0.1M tris-HCl pH 9.5, 5mM $MgCl_2$) (2 x 5 minutes each).
For color development, the wells were incubated at room temperature with a solution of p-nitrophenyl phosphate (5 mg.) in 1 ml. (1M diethanolamine + 2mM $MgCl_2$, pH 8.8). After 10 minutes, the following results were obtained by measuring the color with MicroElisa Autoreader (Dynatech):

| well | 1 | 2 | 3 | 4 | 5 | 6 | 7 (control) |
|---|---|---|---|---|---|---|---|
| O.D. | 1.98 | 1.87 | 1.35 | 1.89 | 1.58 | 1.91 | 0.06 |

EXAMPLE XXXV

Detection of Citrus Exocortis Viroid (CEV) by a Double Hybridization Technique.

This was carried out identically to Example I, with the exceptions that in the first segment each U contains at C-2′ a linker arm of 7 atoms terminated by amino [$-NHCO(CH_2)_5NH_2$] while in the second segment each U contains at C-2′ a linker arm of 7 atoms terminated by biotinamido [$-NHCO(CH_2)_5$-biotinamido].

The following results were obtained:

| well | 1 | 2 | 3 | 4 | 5 | 6 | 7 (control) |
|---|---|---|---|---|---|---|---|
| O.D. | 2.05 | 1.97 | 1.81 | 1.93 | 1.78 | 1.99 | 0.08 |

-----

It will be appreciated by those skilled in the art that many variations and modifications can be made in the practice of the invention as described hereinabove, and consequently the invention is not to be construed as limited merely to what has been particularly described by way of example. Rather, the scope of the invention is limited solely by the claims which follow.

## Claims

1. A chemical compound having the formula (I) wherein <u>either</u> A is a radical of formula $-NHCO-(CH_2)_x-R^3$ and B is a substituted pyrimidine or purine residue, <u>or</u> A is a hydrogen atom and B is a radical of formula (II)

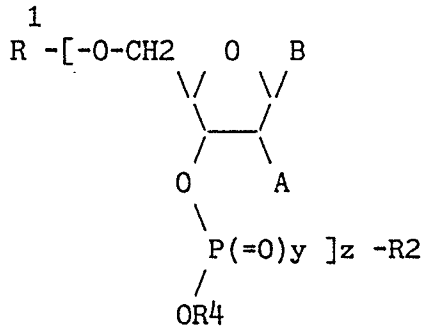

(I)

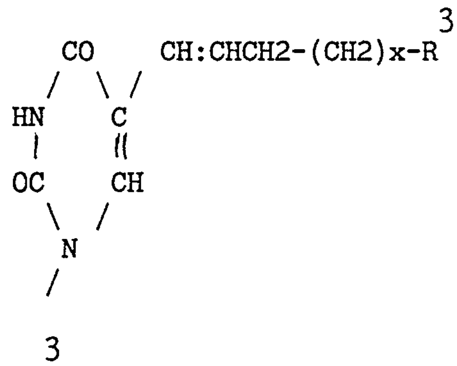

(II)

x is 0-21, $R^3$ is selected from the group consisting of $NH_2$, acylamido, biotinamido, dansyl, alkoxycarbonyl, carboxyl, carbamoyl, substituted carbamoyl, thiocarbamoyl, substituted thiocarbamoyl, ureido, substituted ureido, thioureido, substituted thioureido, a furanoside sugar radical, a pyranoside sugar radical and other reporter groups which can be detected without a requirement for radioactive labelling; $R^1$ is a hydrogen atom, a labile group removable by acid or a radical of formula (III);

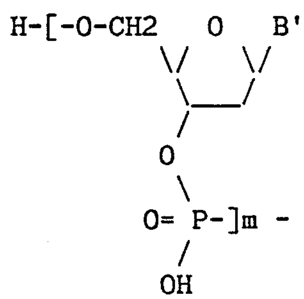

(III)

$R^2$, when $R^1$ is a hydrogen atom or a labile group removable by acid, is halogen, NR′R″ (in which R′ and R″ are the same or different alkyl radicals, or NR′R″ forms a saturated heterocyclic ring) or tetrazolyl, or $R^2$, when $R^1$ is a radical of formula (III), is a radical of formula (IV);

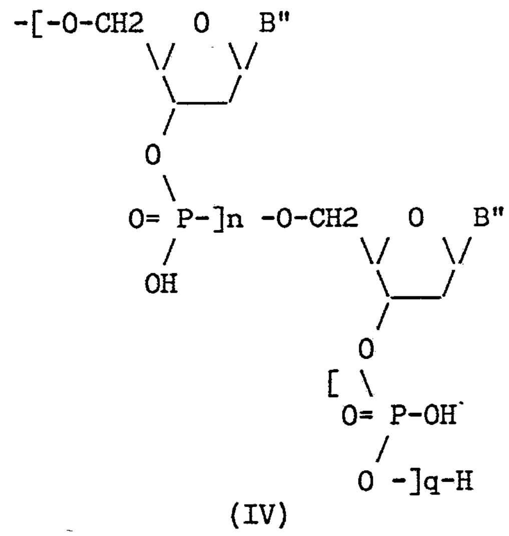

(IV)

$R^4$, when $R^1$ is a hydrogen atom or a labile group removable by acid, is alkyl, or $R^4$, when $R^1$ is a radical of formula (III), is a hydrogen atom; B′ and B″ are each the same or different substituted pyrimidine or purine residues; m and n are each 0 or an integer of 1 to about 1000, q and y are independently 0 or 1, and z is 1 when $R^1$ is a hydrogen atom or a labile group removable by acid, or z is otherwise 1 to about 100; provided that when $R^1$ is a radical of formula (III) and $R^2$ is a radical of formula (IV), then the compound of formula (I) is modified deoxyribonucleic acid (or a corresponding oligonucleotide) in which 1 to about 100 monomeric units in any position(s) have been replaced by the same number of units of formula (V),

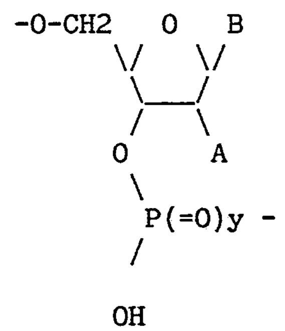

(V)

in which A, B and y are previously defined.

2. A compound of formula (I) according to claim 1, wherein A is a radical of formula $-NHCO-(CH_2)x-R^3$ and B is selected from the group consisting of cytosine, thymidine, adenosine and guanine residues.

3. A compound of formula (I) according to claim 1, wherein A is a hydrogen atom and B is a radical of formula (II) in which x is 3-21.

4. A compound according to either of claims 2 and 3, wherein $R^1$ is a hydroge atom or a labile group removable by acid, $R^4$ is alkyl and z is 1.

5. A compound according to claim 4, wherein y is 0 and $R^2$ is NR′R″ in which R′ and R″ are the same or different alkyl radicals, or NR′R″ forms a saturated heterocyclic ring.

6. A compound according to claim 4, wherein y is 0 and $R^2$ is halogen or tetrazolyl.

7. A compound according to any of claims 4 to 6, wherein $R^1$ is selected from the group consisting of trityl, monomethoxytrityl, dimethoxytrityl and trimethoxytrityl.

8. A compound according to any of claims 2 to 7, wherein $R^3$ is selected from the group consisting of a biotin residue, dansyl, alkanamido such as acetylamino, trifluoroacetylamino, alkoxycarbonyl, a sugar moiety bound as glycoside such as alpha-D-mannopyranoside, or other moieties capable of detection by ELISA or other non-radioactive means, in particular other moieties capable of forming a detectable complex with a polypeptide when the compound is incorporated in a double-stranded duplex formed with a complementary ribonucleic or deoxyribonucleic acid.

9. A compound according to claim 8, wherein said another moiety is selected from the group consisting of dinitrophenyl, fluorescein, rhodamine, nitroiodophenylacetic acid, a steroid, a derivative of luminol or isoluminol, and the residue of any other antigen recognizable by monoclonal or polyclonal antibodies.

10. A compound according to any of claims 2 to 9, wherein x is an integer within the range of from 4 to 8.

11. A process for preparing a compound as defined in claim 2, or in any of claims 4 to 10 as dependent from claim 2, and wherein $R^1$ is a labile group removable by acid, $R^4$ is alkyl and z is 1, which comprises reacting with a reactive ester of $HOOC-(CH_2)x-R^3$, the analogue of the desired compound in which A is primary amino instead of $-NHCO-(CH_2)x-R^3$.

12. A process for preparing a compound as defined in any of claims 1 to 10, and wherein $R^1$ is a labile group removable by acid, $R^4$ is alkyl and z is 1, which comprises reacting with a reagent introducing the labile group $R^1$, the analogue of the desired compound in which $R^1$ is H.

13. A process for preparing a compound as defined in claim 2, or in any of claims 4 to 10 as dependent from claim 2, and wherein $R^1$ is a labile group removable by acid, $R^2$ is Cl, $R^4$ is alkyl and z is 1, which comprises the following steps, namely:

(i) reacting the compound of formula (VI)

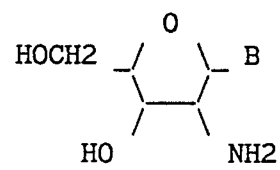

(VI)

with a reactive ester of $HO_2C-(CH_2)x-R^3$, or with the free acid itself in presence of a catalyst, to give the compound of formula (VII),

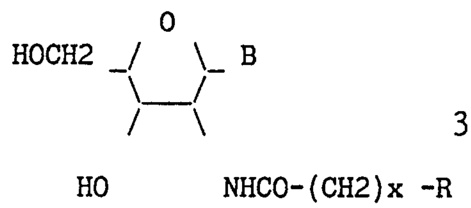

(VII)

(ii) the compound of formula (VII) is reacted with a reagent introducing the labile group $R^1$ to give the compound of formula (VIII)

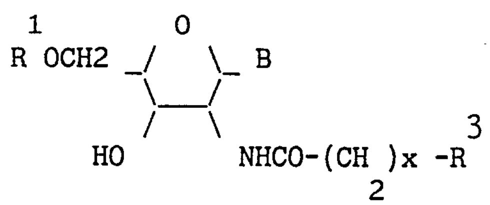

(VIII)

and

(iii) the compound of formula (VIII) is reacted with

alkyl-O-P(=O)y$Cl_2$

in presence of a tertiary amine.

14. A process for preparing a compound as defined in claim 3, or in any of claims 4 to 10 as dependent from claim 3, and wherein $R^1$ is a labile group removable by acid, $R^4$ is alkyl and z is 1, which comprises the following steps, namely:

(a) reacting deoxyuridine with a reagent mercuric compound to form a deoxyuridine-5-mercuric compound;

(b) reacting the latter with a catalyst comprising a palladium compound and with a compound of formula

$CH_2=CHCH_2-(CH_2)x-R^3$, where $R^3$ is

as defined above except primary amino;

(c) treating the mixture with a sulfide reagent and filtering off the insoluble matter comprising mercuric sulfide;

(d) recovering from the filtrate the intermediate of formula (IX);

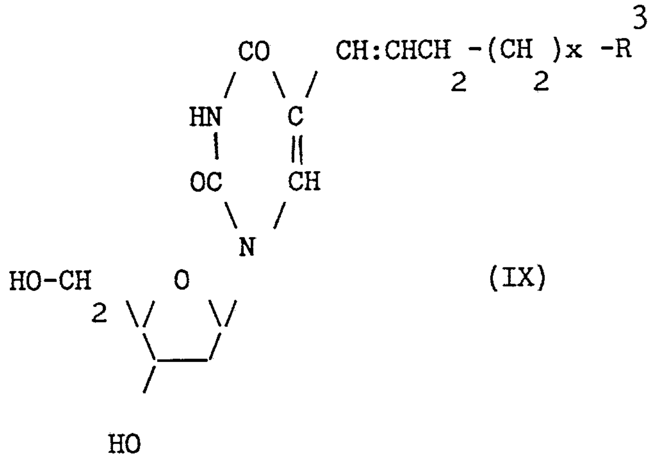

(IX)

(e) reacting compound (IX) with a reagent introducing the labile group $R^1$ in the 5'-position;

(f) reacting the product of the previous step with a reagent of formula alkyl-O-P(X)(=O)y-$R^2$, where X is a leaving group, and optionally carrying out one or both of the following, namely:

(i) where it is desired to obtain the end-product when $R^3$ is primary amino, converting by a method known per se, the product in which $R^3$ is acylamido;

(ii) where it is desired to obtain the end-product when $R^3$ is selected from the group consisting of biotinamido and dansyl, in the case where such compound was not prepared by the process up to step (f), or where it is desired to obtain the end-product when $R^3$ is a different acylamido radical from that prepared by the process up to step (f), reacting the amino-compound of step (i) with a reactant selected from the group consisting of biotinamide, dansyl and an acylating agent which will give the desired different acylamido radical.

15. A process according to claim 14, wherein step (b) is carried out in the presence of an anhydrous cupric compound.

16. A process according to either of claims 14 and 15, wherein said reagent mercuric compound is selected from the group consisting of mercuric alkanecarboxylates and mercuric halides, said palladium compound is selected from the group consisting of alkali metal/palladium halides, alkali metal/palladium alkanecarboxylates and triphenylphosphine palladium halides, and said sulfide reagent is selected from the group consisting of hydrogen sulfide and ammonium and alkali metal sulfides.

17. A process according to any of claims 14 to 16, where in order to obtain a product in which $R^2$ is NR'R" and $R^4$ is methyl or ethyl, a reagent is used in step (f) which has the formula alkyl'-O-P-(X)(=O)y-NR'R", where alkyl' is methyl or ethyl.

18. A compound of formula (I) as set out in claim 1, which is a modified deoxyribonucleic acid (or corresponding oligonucleotide) as defined therein.

19. A compound of formula (I) according to claim 18, wherein A is a radical of formula $-NHCO-(CH_2)x-R^3$ and B is selected from the group consisting of cytosine, thymidine, adenosine and guanine residues.

20. A compound of formula (I) according to claim 18, wherein A is a hydrogen atom and B is a radical of formula (II) in which x is 3-21.

21. A compound of formula (I) according to any of claims 18 to 20, which contains at least two units of formula (V), and wherein 4 to 20 natural nucleotide units separate consecutive units of formula (V).

22. A compound of formula (I) according to any of claims 18 to 21, wherein $R^3$ is selected from the group consisting of a biotin residue, dansyl, alkanamido such as acetylamino, trifluoroacetylamino, alkoxycarbonyl, a sugar moiety bound as glycoside such as alpha-D-mannopyranoside, or other moieties capable of detection by ELISA or other non-radioactive means, in particular other moieties capable of forming a detectable complex with a polypeptide when the compound is incorporated in a double-stranded duplex formed with a complementary ribonucleic or deoxyribonucleic acid.

23. A compound of formula (I) according to claim 22, wherein said another moiety is selected from the group consisting of dinitrophenyl, fluorescein, rhodamine, nitroiodophenylacetic acid, a steroid, a derivative of luminol or isoluminol, and the residue of any other antigen recognizable by monoclonal or polyclonal antibodies.

24. A compound of formula (I) according to any of claims 18 to 23, wherein x is an integer within the range of from 4 to 8.

25. A process for preparing a compound of formula (I) as defined in any of claims 18 to 24, wherein a corresponding compound of formula (I) as defined in any of claims 4 to 7 (except that $R^3$ may not be primary amino) is reacted with a supported and protected 2'-deoxynucleotide or oligo-2'-deoxynucleotide which has been synthesized by a method known per se, and if and when desired, any one or more of the following further steps is carried out, namely:

(i) the conversion of acylamido to amino by a method known per se, and/or

(ii) the reaction of amino formed in step (i) with biotinamide or with dansyl, or with an acylating agent which will give the desired value for $R^3$ where this is acylamido in the product, or with any other compound of structure such that in the product it has become converted to a moiety capable of forming a detectable complex with a polypeptide when the compound is incorporated into a double-stranded duplex formed with a complementary ribonucleic or deoxyribonucleic acid, and/or

(iii) the oxidation of any phosphite ester residues present to phosphate ester residues.

26. A compound of formula (I) as defined in any of claims 18 to 24, which has been prepared according to the process of claim 25.

27. A compound of formula (I) as defined in any of claims 18 to 24 or 26, where $y = 1$.

28. A duplex strand derived from DNA characterized by the presence of:

(i) a double stranded region of from 10 to about 1000 complementary base pairs;

(ii) a top single strand comprising a compound of formula (I) as defined in claim 27;

(iii) a bottom single strand of natural complementary DNA.

29. A duplex strand according to claim 28 wherein in the top single strand at least two units of formula (V) are present, and any two consecutive such units are separated from each other by about 4 to 20 units of the naturally occurring nucleotides.

30. A process in which one or more duplex strands as defined in either of claims 28 and 29 is subjected to an annealing reaction.

31. A method for the detection of a target which is a bacterium, fungus, virus, viroid or other source of nucleic acids, which comprises the steps of hybridization with at least one polymeric or oligomeric phosphate of formula (I) as defined in claim 27, and detecting the group $R^3$ in the thus-formed hybrid or hybrids, by a non-radioactive method.

32. A method according to claim 31, wherein following the hybridization step, the hybrid is removed from the reaction mixture and complexed with a polypeptide appropriate for the particular $R^3$ group present, and the polypeptide is detected, thereby enabling identification and estimation of the source of nucleic acids to be effected.

33. A method according to claim 31, wherein $R^3$ is a biotin residue, and the polypeptide is avidine to which is attached a residue selected from the group consisting of fluorescein, rhodamine, peroxidase and alkaline phosphatase residues.

34. A method according to claim 32, wherein $R^3$ is a biotin residue, and the polypeptide is streptavidine to which is attached a residue selected from the group consisting of fluorescein, rhodamine, peroxidase and alkaline phosphatase residues.

35. A method according to claim 32, wherein $R^3$ is the residue of a steroid or of any other antigen recognizable by monoclonal or polyclonal antibodies and the polypeptide is comprised by monoclonal or polyclonal antibodies which are characterized by techniques known per se.

36. A method according to claim 35 wherein $R^3$ is the residue of a steroid which is selected from the group consisting of testosterone, progesterone, estradiol and their derivatives.

37. A method according to claim 32 wherein $R^3$ is the dinitrophenyl group or the residue of nitroiodophenylacetic acid.

38. A method according to claim 31 which is utilized for the detection of a plant virus or viroid, and which comprises the steps of:
grinding at least one leaf disk of a plant to be detected in presence of an extraction buffer;
centrifuging the extract;
heating the supernatant to an elevated temperature;
spotting the heated supernatant on nitrocellulose paper presoaked in aqueous NaCl and sodium citrate or the chemical equivalents thereof;
pouring a solution of the complementary phosphate defined in claim 27 containing a detectable group $R^3$ onto the spotted nitrocellulose paper;
washing with saline solution to remove unhybridized reagent; and
detecting the group $R^3$ in a manner known per se.

39. A method according to claim 31, which comprises hybridizing the target with two species of the said phosphate and separating and detecting the thus-formed hybrid(s).

40. A method according to claim 39, which comprises the steps of:

(i) separating the target to be identified or estimated;

(ii) hybridizing the target with said two species, namely one in which $R^3$ comprises a biotin residue and one in which $R^3$ comprises a steroid residue;

(iii) separating the hybrid(s) using microtiter plates and/or immunobeads and/or sticks coated with monoclonal or polyclonal antibodies against the steroid or other antigen, followed by washing out the unhybridized target and/or unreacted reagent;

(iv) detection of the hybridized target in which $R^3$ comprises a biotin residue by means of reagents selected from the group consisting of streptavidine-peroxidase, streptavidine-alkaline phosphatase, streptavidine/fluoroescein, streptavidine/rhodamine, streptavidine/dansyl and streptavidine/isoluminol derivatives; and

(v) detection of the enzyme residue by means selected from ELISA, chemiluminescence and fluorescence.

41. A method according to claim 31, which comprises the steps of:

(i) reacting one such phosphate as defined in claim 27, which contains a suitably polypeptide-reactive terminal group $R^3$, with a polypeptide containing a plurality of radicals reactive with said terminal $R^3$ group selected from the group consisting of free $NH_2$ and $CO_2H$ radicals, so as to form covalent bonding between said compound and the polypeptide;

(ii) removing unreacted said compound;

(iii) hybridizing consecutively or simultaneously the target with the polypeptide reaction product thus prepared and with a separately prepared phosphate as defined in claim 27, containing a detectable $R^3$ ($R^{3'}$) radical;

(iv) removing unreacted components; and

(v) detecting radical $R^{3'}$ in the doubly-hybridized target.

42. A method according to claim 41, wherein the detectable $R^{3'}$ radical is a biotin residue, and in stage (v) this residue is detected in the doubly-hybridized target by means of streptavidine-enzyme or avidine-enzyme, whereby the enzyme is detected by means of a method selected from the group consisting of ELISA, chemiluminescence and fluorescence.

43. A method according to claim 42 wherein the enzyme in the streptavidine-enzyme or avidine-enzyme entity is selected from the group consisting of peroxidase and alkaline phosphatase.

44. A method according to claim 41, wherein the detectable $R^{3'}$ radical is a biotin residue, and in stage (v) this residue is detected in the doubly-hybridized target by means of streptavidine/dye or avidine/dye, and the dye is selected from the group consisting of fluorescein, rhodamine, dansyl and isoluminol derivatives.

45. A method according to claim 41, wherein the detectable $R^{3'}$radical is selected from the group consisting of dansyl, nitrophenyl, the residue of fluorescein, rhodamine, nitroiodophenylacetic acid, and of a steroid, a derivative of luminol or isoluminol, and the residue of any other antigen recognizable by monoclonal or polyclonal antibodies, and in stage (v) this group is detected by a method known per se.

46. A method according to any of claims 41 to 45, wherein the initial polypeptide containing a plurality of reactive radicals, to be reacted in stage (i), is first coated onto microtiter plates, beads or sticks.

47. A method according to any of claims 41 to 46, wherein at least one of steps (ii) and (iv) is effected by washing.

48. A method according to any of claims 31 to 37 or 39 to 47, where the hybridization is effected in solution.

49. A method according to any of claims 31 to 48, which is applied to detect a species selected from the group consisting of avocado sunblotch viroid, tobacco mosaic virus and citrus exocortis viroid.

50. A duplex strand according to claim 28, wherein said a double stranded region contains from about 20 to about 200 complementary base pairs.

51. A duplex strand according to either of claims 29 and 49, wherein in the top single strand at least two units of formula (V) are present, and any two consecutive such units are separated from each other by 4 to 6 units of the naturally occurring nucleotides.

European Patent Office

# EUROPEAN SEARCH REPORT

Application number
EP 86 30 9090

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | No relevant documents have been disclosed.<br>----- | | C 07 H 19/06<br>C 07 H 19/16<br>C 07 H 19/20<br>C 07 H 21/00 |
| | | | TECHNICAL FIELDS SEARCHED (Int. Cl.4) |
| | | | C 07 H 21/00<br>C 07 H 19/00 |

~~The present search report has been drawn up for all claims~~

| Place of search | Date of completion of the search | Examiner |
|---|---|---|
| The Hague | 16-07-1987 | VAN BIJLEN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

## X LACK OF UNITY OF INVENTION

The Search Division considers that the present European patent application does not comply with the requirement of unity of invention and relates to several inventions or groups of inventions,

namely:

1) Claims 1-51 partially:
So far as related to formula (I) wherein
A = $-NHCO-(CH_2)_x-R_3$ and
B = a substituted pyrimidine or purine residue
(i.e. 2'-substituted-2'-deoxynucleotides).

2) Claims 1-51 partially:
So far as related to formula (I) wherein
A = H and
B = formula (II)
(i.e. 5-substituted-2'-deoxyuridines)

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims: 1-51 partially (point 1)